# EUROPEAN PATENT APPLICATION

(11) **EP 4 742 266 A1**
(43) Date of publication of application: **13.05.2026**
(21) Application number: 23962568.4
(22) Date of filing: 26.12.2023
(51) Int. Cl.: G16H 40/60, G16H 80/00, G08B 23/00, G08B 25/00, A61B 5/00

(54) **MEDICAL DEVICE AND INFORMATION DISPLAY METHOD THEREFOR**

(71) Applicant: Shenzhen Mindray Bio-Medical Electronics Co., Ltd., Shenzhen, Guangdong 518057 (CN)
(72) Inventor: ZHANG, Jianhui, Shenzhen, Guangdong 518057 (CN); YUAN, Weiwei, Shenzhen, Guangdong 518057 (CN); LIU, Yan, Shenzhen, Guangdong 518057 (CN); DAI, Weiwei, Shenzhen, Guangdong 518057 (CN)
(74) Representative: KIPA AB
(86) International application number: PCT/CN2023/142062
(87) International publication number: WO 2025/137874

(57) **Abstract**

A medical device and an information display method therefor. The medical device comprises a processor and a display. The processor is used for acquiring patient information of a plurality of patients. The display is used for presenting a plurality of display regions on a same display interface, each display region presenting patient information of one patient, and the patient information further comprising alarm information associated with a monitoring information abnormality and/or a medical device abnormality. For at least one display region in which alarm information is present, before a confirmation operation for the alarm information in the display region is received, the alarm information is displayed in the display region in a first display mode, and an additional prompt element is displayed to highlight the presence of current unconfirmed alarm information. After the confirmation operation is received, at least part of the alarm information is displayed in the display region in a second display mode, and the additional prompt element is removed. Compared to the alarm information displayed in the first display mode, the alarm information displayed in the second display mode presents a visual effect of reduced display.

## Description

### TECHNICAL FIELD

The present disclosure relates generally to the technical field of hospital devices, and more particularly to a hospital device and an information display method for the hospital device.

### BACKGROUND

During clinical treatment, medical staff usually monitor multiple patients simultaneously through a hospital device such as a central station. The central station usually includes a host computer (a processor) and a display. A data communication connection is established between the host computer and a medical device such as a patient monitor, to obtain patient information. This information is then displayed on the display to monitor the patient.

In a current display scheme, when the patient monitoring information is abnormal or the medical device used by the patient is abnormal, alarm information associated with the patient will be displayed on the display. Emergency alarm requires the medical staff to take treatment measures immediately upon knowing it. If the medical staff cannot be notified promptly, this could lead to serious consequences. However, because the information of multiple patients is displayed on the display, a volume of the displayed information is large, and it may be difficult for the medical staff to pay attention to the emergency alarm immediately, which may lead to serious consequences.

In addition, after the medical staff sees the alarm information, they will perform a confirmation operation for the alarm information, indicating that the medical staff is aware of the alarm and will take relevant measures in time to deal with related abnormalities. However, in the current display scheme, after the medical staff performs the confirmation operation for the alarm information, the alarm information is still prominently displayed until a cause of the alarm is resolved, which will disturb the attention of the medical staff and make it difficult for them to notice the alarm information that requires more attention but has not been confirmed.

### SUMMARY

In order to solve at least one of the above problems, the present disclosure provides a hospital device and an information display method for the hospital device.

According to an aspect of the present disclosure, there is provided a hospital device including a processor and a display. The processor is configured to obtain patient information for a plurality of patients, the display is configured to present a plurality of display regions on a same display interface, and each of the plurality of display regions presents the patient information for a respective patient. The patient information includes at least patient monitoring information, and when the patient monitoring information is abnormal or a medical device used by the patient is abnormal, the patient information further includes alarm information associated with the monitoring information being abnormal and/or the medical device being abnormal. For at least one display region where the alarm information is present, the alarm information is displayed in the display region in a first display mode before receiving a confirmation operation for the alarm information in the display region, and an additional prompt element is additionally displayed in the display region to highlight a presence of an unconfirmed alarm information. The additional prompt element is displayed in a display mode different from the first display mode, and/or a display region occupied by the additional prompt element is different from the display region occupied by the alarm information. At least a portion of the alarm information is displayed in the display region in a second display mode after receiving the confirmation operation, and the additional prompt element is eliminated. The alarm information displayed in the second display mode exhibits a weakened visual effect relative to the alarm information displayed in the first display mode.

According to another aspect of the present disclosure, there is provided an information display method for a hospital device, and the method includes the following operations. Patient information for a plurality of patients is obtained; and a plurality of display regions are presented on a same display interface, and each of the plurality of display regions presents the patient information for a respective patient. The patient information includes at least patient monitoring information, and when the patient monitoring information is abnormal or a medical device used by the patient is abnormal, the patient information further includes alarm information associated with the monitoring information being abnormal and/or the medical device being abnormal. For at least one display region for the alarm information, the alarm information is displayed in the display region in a first display mode before receiving a confirmation operation for the alarm information in the display region, and an additional prompt element is additionally displayed in the display region to highlight a presence of an unconfirmed alarm information. The additional prompt element is displayed in a display mode different from the first display mode, and/or a display region occupied by the additional prompt element is different from the display region occupied by the alarm information. At least a portion of the alarm information is displayed in the display region in a second display mode after receiving the confirmation operation, and the additional prompt element is eliminated. The alarm information displayed in the second display mode exhibits a weakened visual effect relative to the alarm information displayed in the first display mode.

According to yet another aspect of the present disclosure, there is provided a hospital device including a processor and a display. The processor is configured to obtain patient information for a plurality of patients, the display is configured to present a plurality of display regions on a same display interface, and each of the plurality of display regions presents the patient information for a respective patient. The patient information includes at least patient monitoring information, and when the patient monitoring information is abnormal or a medical device used by the patient is abnormal, the patient information further includes alarm information associated with the monitoring information being abnormal and/or the medical device being abnormal. When the processor obtains the alarm information associated with the monitoring information being abnormal and/or the medical device being abnormal, the display is configured to: display the alarm information in a third sub-region in a display region for the patient corresponding to the alarm information, and additionally display an additional prompt element corresponding to the alarm information in at least one portion of the region outside the third sub-region or in the display region in its entirety where the alarm information is present. Respective additional prompt elements corresponding to at least two types of alarm information are different.

According to yet another aspect of the present disclosure, there is provided an information display method for a hospital device, and the method includes the following operations. Patient information for a plurality of patients is obtained; a plurality of display regions are presented on a same display interface, and each of the plurality of display regions presents the patient information for a respective patient. The patient information includes at least patient monitoring information, and when the patient monitoring information is abnormal or a medical device used by the patient is abnormal, the patient information further includes alarm information associated with the monitoring information being abnormal and/or the medical device being abnormal. When the alarm information, associated with the monitoring information being abnormal and/or the medical device being abnormal, is obtained, the alarm information is displayed in a third sub-region in a display region for the patient corresponding to the alarm information, and an additional prompt element corresponding to the alarm information is additionally displayed in at least one portion of the region outside the third sub-region or in the display region in its entirety where the alarm information is present. Respective additional prompt elements corresponding to at least two types of alarm information are different.

The hospital device and the information display method therefor in the present disclosure display the patient information for a plurality of patients on the same display interface. Before receiving the confirmation operation for the alarm information, the alarm information is displayed in a relatively prominent first display mode, and the additional prompt elements is additionally displayed, which is beneficial for medical staff to notice the alarm information and process it in time. After receiving the confirmation operation for the alarm information, the alarm information is displayed in a less prominent and visually weakened second display mode and the previously displayed additional prompt element is eliminated, to prevent the alarm information from being too conspicuous and distracting the attention of the medical staff from other unconfirmed alarm information, thereby enabling the medical staff to more easily notice the other unconfirmed alarm information.

### BRIEF DESCRIPTION OF THE DRAWINGS

FIG. 1 illustrates an example diagram of a hospital device and a schematic display interface of the hospital device according to an embodiment of the present disclosure.
FIG. 2 illustrates a schematic diagram of a display region for each patient on a display interface of a hospital device according to an embodiment of the present disclosure.
FIG. 3 illustrates an example diagram of alarm information displayed in a first display mode and a second display mode respectively on a display interface of a hospital device before and after receiving a confirmation operation according to an embodiment of the present disclosure.
FIG. 4 illustrates another example diagram of alarm information displayed in a first display mode and a second display mode respectively on a display interface of a hospital device before and after receiving a confirmation operation according to an embodiment of the present disclosure.
FIG. 5 illustrates an example diagram of displaying and eliminating an additional prompt element on a hospital device according to an embodiment of the present disclosure.
FIG. 6 illustrates another example diagram of displaying and eliminating an additional prompt element on a hospital device according to an embodiment of the present disclosure.
FIG. 7 illustrates yet another example diagram of displaying and eliminating an additional prompt element on a hospital device according to an embodiment of the present disclosure.
FIG. 8 illustrates yet another example diagram of displaying and eliminating an additional prompt element on a hospital device according to an embodiment of the present disclosure.
FIG. 9 illustrates yet another example diagram of displaying and eliminating an additional prompt element on a hospital device according to an embodiment of the present disclosure.
FIG. 10 illustrates yet another example diagram of displaying and eliminating an additional prompt element on a hospital device according to an embodiment of the present disclosure.
FIG. 11 illustrates yet another example diagram of displaying and eliminating an additional prompt element on a hospital device according to an embodiment of the present disclosure.
FIG. 12 illustrates a schematic diagram of a more specific example of an interface change before and after receiving a confirmation operation for alarm information on a display interface of a hospital device according to an embodiment of the present disclosure.
FIG. 13 illustrates a schematic flowchart of an information display method for a hospital device according to an embodiment of the present disclosure.
FIG. 14 illustrates an example diagram of a hospital device and a schematic display interface for the hospital device according to another embodiment of the present disclosure.
FIG. 15 illustrates a schematic flowchart of an information display method for a hospital device according to another embodiment of the present disclosure.

### DETAILED DESCRIPTION

To make objects, technical solutions, and advantages of the present disclosure more apparent, exemplary embodiments according to the present disclosure will be described in detail below with reference to the accompanying drawings. It is apparent that the described embodiments are only part of the embodiments of the present disclosure, and not all of the embodiments of the present disclosure. It should be understood that the present disclosure is not limited by the exemplary embodiments described herein. All other embodiments obtained by those skilled in the art based on the embodiments of the present disclosure described herein without creative effort should fall within the scope of protection of the present disclosure.

In the following description, numerous specific details are given in order to provide a more thorough understanding of the present disclosure. However, it will be apparent to those skilled in the art that the present disclosure may be practiced without one or more of these details. In other examples, in order to avoid confusion with the present disclosure, some technical features well known in the art have not been described.

It is to be understood that the present disclosure may be implemented in various forms and should not be construed as limited to the embodiments presented herein. On the contrary, the provision of these embodiments would render the disclosure thorough and complete, and fully convey the scope of the present disclosure to those skilled in the art.

The terminology used herein is for the purpose of describing specific embodiments only and do not constitute a limitation of the present disclosure. As used herein, singular forms such as "a", "an", and "the" are also intended to include plural forms unless the context clearly dictates otherwise. It should also be understood that the terms "comprise" and/or "include", when used in the description, establish the presence of features, integers, steps, operations, elements, and/or components, but do not preclude the presence or addition of one or more other features, integers, steps, operations, elements, components, and/or groups. As used herein, the term "and/or" includes any and all combinations of the relevant listed items.

To thoroughly understand the present disclosure, detailed steps and structures will be proposed in the following description in order to explain the technical solutions proposed in the present disclosure. Preferred embodiments of the present disclosure are described in detail below, however, the present disclosure may have other embodiments in addition to these detailed descriptions.

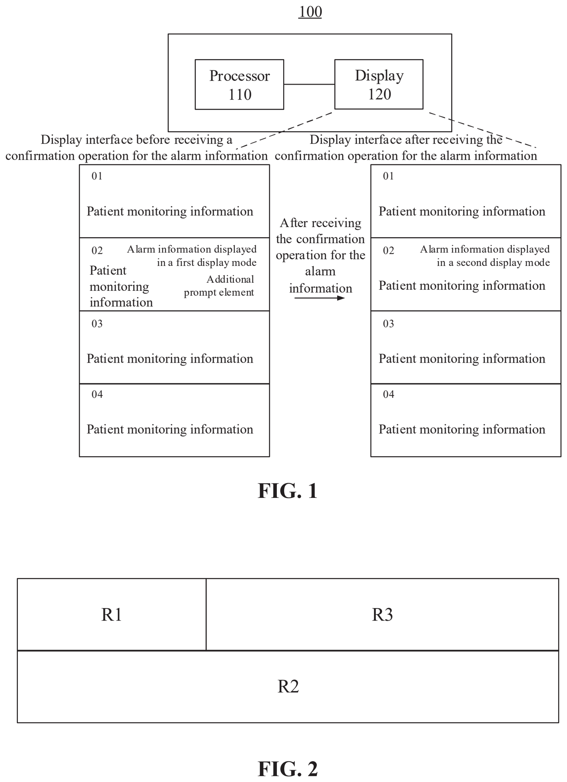

FIG. 1 illustrates an example diagram of a hospital device 100 and a schematic display interface for the hospital device according to an embodiment of the present disclosure. As shown in FIG. 1, the hospital device 100 includes a processor 110 and a display 120.

The processor 110 is configured to obtain patient information for a plurality of patients. For example, the hospital device 100 is communicatively connected with a medical device (such as a monitor, a ventilator, etc.) used by each of the plurality of patients, the processor 110 may obtain monitoring information for each of the plurality of patients from these monitoring devices, and display the monitoring information on the display 120. Further, when the patient monitoring information is abnormal or the medical device used by the patient is abnormal, alarm information, associated with the monitoring information being abnormal and/or the medical device being abnormal, may be displayed on the display 120, and the alarm information is also one type of patient information.

The display 120 is configured to present a plurality of display regions, such as display region 01, display region 02, display region 03, and display region 04 shown in FIG. 1, on a same display interface, and each of the plurality of display regions presents patient information for a respective patient. For the sake of brevity, FIG. 1 illustrates only four display regions on the display interface for presenting patient information for four patients, which is merely an example. In practical applications, the number of display regions, and an arrangement of the number of rows and the number of columns included in the display interface may depend on the number of patients to be monitored, and the display interface may include one column and multiple rows, two columns and multiple rows, multiple columns and multiple rows, etc.

The patient information presented in each display region includes at least the patient monitoring information described above. When the patient monitoring information is abnormal or the medical device used by the patient is abnormal, the patient information further includes alarm information associated with the monitoring information being abnormal and/or the medical device being abnormal.

For at least one display region where the alarm information is present (in FIG. 1, for example, the display region 02 is a display region where the alarm information is present, and an actual number of display regions where the alarm information is present may be one or more), the alarm information is displayed in the display region in a first display mode before receiving a confirmation operation for the alarm information in the display region, and an additional prompt element is additionally displayed to highlight a presence of an unconfirmed alarm information. The additional prompt element is displayed in a display mode different from the first display mode, and/or a display region occupied by the additional prompt element is different from the display region occupied by the alarm information. At least a portion of the alarm information is displayed in the display region in a second display mode after receiving the confirmation operation, and the additional prompt element is eliminated. The alarm information displayed in the second display mode exhibits a weakened visual effect relative to the alarm information displayed in the first display mode.

In the embodiment of the present disclosure, the display 120 of the hospital device 100 displays the patient information for a plurality of patients on the same display interface. When a patient exhibits abnormal conditions (the patient monitoring information is abnormal or the medical device used by the patient is abnormal), the display region for the patient will display corresponding alarm information. The alarm information is displayed in different display modes before and after receiving the confirmation operation. Specifically, before receiving the confirmation operation, the alarm information is displayed in a relatively prominent first display mode, which is beneficial for the medical staff to notice the alarm information and process the alarm information in time. Further, before receiving the confirmation operation, an additional prompt element is additionally displayed to further highlight the current presence of the unconfirmed alarm information on the basis of the prominent first display mode, to enable the medical staff to more easily notice the newly occurring and unconfirmed alarm information and process it in time. Further, after the medical staff confirms the alarm information, since they are already aware of the alarm and have carried out the confirmation operation, the medical staff will normally perform corresponding processing according to specific content of the alarm information. Because the processing takes time, the alarm information will still be displayed until the abnormal problem triggering the alarm is resolved. At this time, the alarm information is no longer displayed in the previous prominent first display mode. Instead, the alarm information is displayed in a less prominent second display mode with a weaker visual effect compared to the first display mode, to prevent the alarm information from being too conspicuous and affecting the attention of the medical staff to other unconfirmed alarm information, thereby enabling the medical staff to more easily notice other unconfirmed alarm information. In addition, after receiving the confirmation operation, the additional prompt element previously displayed is also eliminated, so that it is easier to distinguish between the confirmed and unconfirmed alarm information, and further avoids affecting the attention of the medical staff to other unconfirmed alarm information. Such a display scheme is particularly important for displaying information of multiple patients on the same interface, because there may be a situation where multiple patients are abnormal and multiple pieces of alarm information are displayed in different display regions in the same interface. Such a display mode is more conducive to the medical staff paying attention to newly occurring and unconfirmed alarm information, avoiding an emergency alarm from being overlooked due to numerous alarm information on the interface, and thus avoiding serious consequences.

In an embodiment of the present disclosure, the patient information further includes patient identification information, and each of the plurality of display regions includes a first sub-region for displaying the patient identification information, a second sub-region for displaying the patient monitoring information, and a third sub-region for displaying the alarm information, which will be described below in conjunction with FIG. 2 as an example.

FIG. 2 illustrates a schematic diagram of a display region for each patient on a display interface of a hospital device according to an embodiment of the present disclosure, that is, FIG. 2 shows any one of the display region 01 to the display region 04 in FIG. 1. As shown in FIG. 2, the display region for the patient may include a first sub-region R1, a second sub-region R2, and a third sub-region R3. The first sub-region R1 is used for displaying the patient identification information (such as at least one of a name, a bed number, a medical record number of the patient and the like). The patient monitoring information may be more effectively correlated with the patient based on the information displayed in the first sub-region. The second sub-region R2 is used for displaying the patient monitoring information of the patient, such as an electrocardiographic waveform, a blood oxygen value, and the like. The third sub-region R3 is used for displaying the alarm information. When there is no abnormality in the patient, the third sub-region R3 does not display any information.

At least one of the first sub-region R1, the second sub-region R2, and the third sub-region R3 may be further subdivided into a plurality of regions according to the number, type, and the like of the displayed information. For example, the alarm information may generally include physiological alarm information and technical alarm information. The physiological alarm information is usually an alarm for abnormal patient monitoring information, such as an excessively high blood oxygen value of the patient. The technical alarm information usually indicates an abnormality in the medical device used by the patient, such as low power of the monitoring device used by the patient, etc. Therefore, the third sub-region R3 may be further divided into two sub-units, one sub-unit displays the technical alarm information and the other sub-unit displays the physiological alarm information. Generally, due to a limitation of a size of the display and a need to display the information for a plurality of patients in the display interface of the display, a size of the display region for each patient is limited. When there are multiple pieces of physiological alarm information, the physiological alarm information with the highest level is usually displayed, while the physiological alarm information with a next-highest or lower level is hidden and only displayed when the user performs a certain operation. Similarly, this applies to the technical alarm information. In addition, due to the foregoing factors such as the size of the display and the number of patients, the third sub-region R3 may not be divided into the above two sub-units, but only one sub-unit that displays the alarm information with the highest level among the physiological alarm information and the technical alarm information.

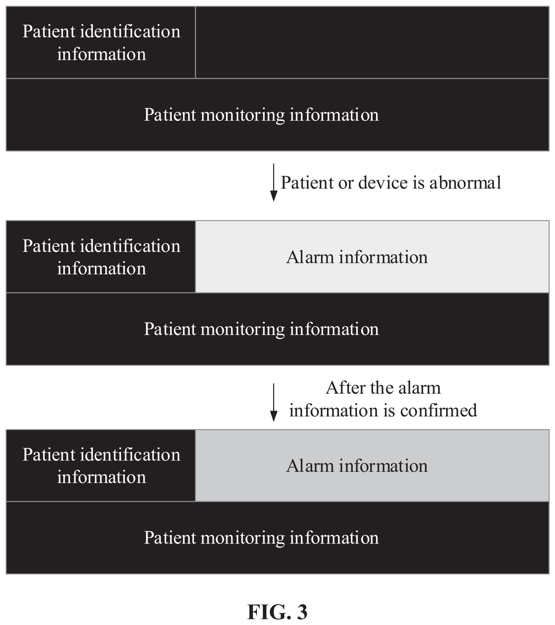

In an embodiment of the present disclosure, the first display mode (that is, the display mode before the alarm information is confirmed) described above may include that: a background color of the third sub-region is changed from an original background color to a first color; and the second display mode (that is, the display mode after the alarm information is confirmed) described above may include that: the background color of the third sub-region is changed from the first color to a second color. The second color is obtained by reducing a brightness or transparency of the first color. This will be described below with reference to FIG. 3.

FIG. 3 illustrates an example diagram of alarm information displayed in a first display mode and a second display mode respectively on a display interface of a hospital device before and after receiving a confirmation operation according to an embodiment of the present disclosure. As shown in FIG. 3, for example, before the alarm information is displayed, the background color of the third sub-region is black (that is, the original background color), and when the alarm information is displayed, the background color of the third sub-region is changed, for example, the original black color is changed to a more conspicuous first color (such as red or yellow), in addition to displaying the specific content of the alarm information (for example, text information such as high blood oxygen value) in the third sub-region. In this way, the alarm information may be more highlighted to attract the attention of the medical staff. It should be noted that the drawings of the present disclosure are gray-scale images, so the original color may not be seen, and the interface color may be set according to the description in the present disclosure in a practical application.

Further, the first color may be a color capable of reflecting a level of the alarm information. For example, it is assumed that the alarm information is categorized as high-level alarm information and medium-level alarm information. The color corresponding to the high-level alarm information is red, and the color corresponding to the medium-level alarm information is yellow. Then, when the alarm information is the high-level alarm information, the high-level alarm information may be displayed in the third sub-region, and at the same time, the background color of the third sub-region may be changed from black to red, to enable the medical staff to immediately know that a high-level alarm has occurred upon they see the background color. Similarly, when the alarm information is the medium-level alarm information, the medium-level alarm information may be displayed in the third sub-region, and at the same time, the background color of the third sub-region may be changed from black to yellow, to enable the medical staff to immediately know that a medium-level alarm has occurred upon they see the background color. It should be understood that the above-described colors are all exemplary, and in practical applications, the above-described colors may be set according to needs, and are not limited to the colors in the above-described examples.

With continued reference to FIG. 3, after the alarm information is confirmed, the background color of the third sub-region changes from the first color to the second color. It is assumed that the first color is red, the second color may be a dark red that becomes dim after being lowered in brightness, or a light red that becomes less prominent than before because it becomes more transparent after being lowered in transparency. In this way, the confirmed alarm information is displayed in a weakened manner relative to the alarm information before confirmation, and its interference to the medical staff will be greatly reduced.

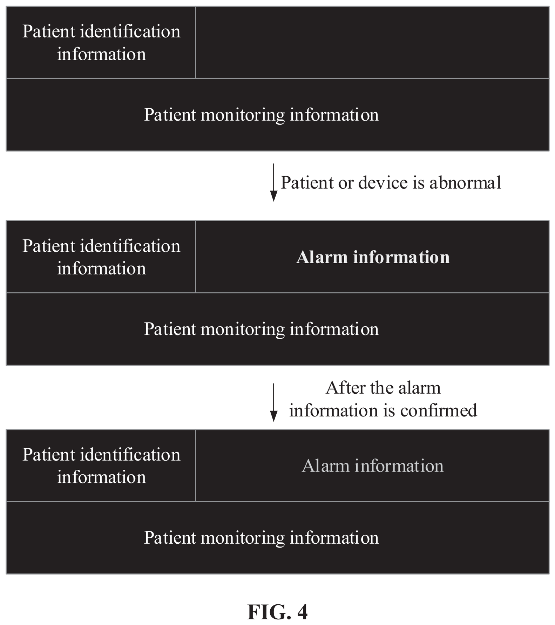

In another embodiment of the present disclosure, the first display mode (that is, the display mode before the alarm information is confirmed) described above may include: displaying the alarm information with a first text attribute. The second display mode (that is, the display mode after the alarm information is confirmed) described above may include: displaying the alarm information with a second text attribute. The first text attribute has a higher brightness of a text color and/or a bolder text font than the second text attribute. This will be described below with reference to FIG. 4.

FIG. 4 illustrates another example diagram of alarm information displayed in a first display mode and a second display mode respectively on a display interface of a hospital device before and after receiving a confirmation operation according to an embodiment of the present disclosure. As shown in FIG. 4, for example, before the alarm information is confirmed, the alarm information is displayed in bold red font, so that the alarm information may be more highlighted to attract the attention of the medical staff. After the alarm information is confirmed, the alarm information is displayed in a dark red which becomes dim after the brightness of red is reduced, or a light red which becomes less prominent than before because the transparency of red is reduced and it becomes more transparent, and a non-bold font, so that the confirmed alarm information is displayed in a weakened manner relative to the alarm information before confirmation, and its interference to the medical staff will be greatly reduced.

Here, the text color of the first text attribute may be a color capable of reflecting the level of the alarm information. For example, it is assumed that the alarm information is categorized as high-level alarm information and medium-level alarm information. The color corresponding to the high-level alarm information is red, and the color corresponding to the medium-level alarm information is yellow. Then, when the alarm information is the high-level alarm information, the high-level alarm information may be displayed in the third sub-region in red font, to enable the medical staff to immediately know that a high-level alarm has occurred upon they see the font color. Similarly, when the alarm information is the medium-level alarm information, the medium-level alarm information may be displayed in the third sub-region in yellow font, to enable the medical staff to immediately know that a medium-level alarm has occurred upon they see the font color. It should be understood that the above-described colors are all exemplary, and in practical applications, the above-described colors may be set according to needs, and are not limited to the colors in the above-described examples.

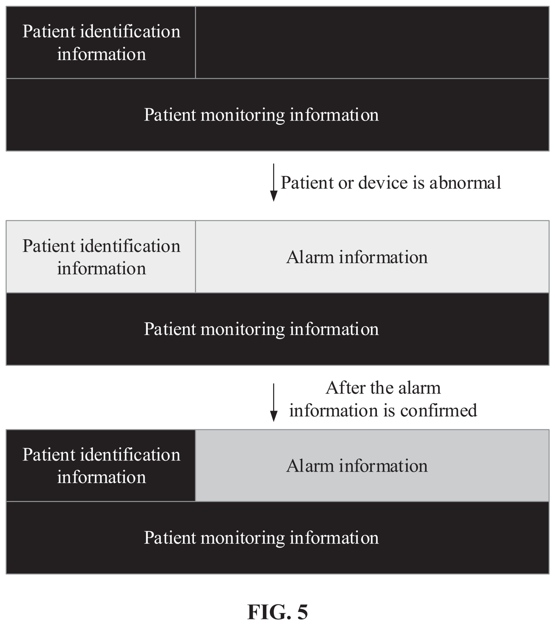

In an embodiment of the present disclosure, the above-described operation of displaying the additional prompt element while displaying the alarm information in the first display mode may include: changing a background color of at least one portion of the first sub-region (such as the first sub-region R1 shown in FIG. 2) and/or at least one portion of the second sub-region (such as the second sub-region R2 shown in FIG. 2) from an original background color to a third color. Accordingly, the operation of eliminating the additional prompt element while displaying the alarm information in the second display mode may include: changing the background color of the at least one portion of the first sub-region (such as the first sub-region R1 shown in FIG. 2) and/or the at least one portion of the second sub-region (such as the second sub-region R2 shown in FIG. 2) from the third color back to the original background color. This will be described below with reference to FIG. 5 to FIG. 7.

FIG. 5 illustrates an example diagram of displaying an additional prompt element on a hospital device according to an embodiment of the present disclosure, in which a schematic diagram of displaying the additional prompt element in a first sub-region is illustrated, and in this example, it is illustrated that the background color of the entire first sub-region is changed. It should be understood that the background color of a portion of the first sub-region may also be changed. As shown in FIG. 5, for example, before the alarm information is displayed, the background colors of the first sub-region, the second sub-region, and the third sub-region are all black (that is, the original background color), and when the alarm information is displayed, the background color of the third sub-region is changed, for example, from the original black to a more conspicuous first color (such as red or yellow), in addition to displaying the specific content of the alarm information (for example, text information such as high blood oxygen value) in the third sub-region. In this way, the alarm information may be more highlighted to attract the attention of the medical staff. In addition, the background color of the first sub-region is changed to a third color (for example, red or yellow, etc.) to further highlight which patient is currently triggering the alarm. The first sub-region is generally used to display patient identification information, and the change of the background color may further highlight the patient experiencing an anomaly. Moreover, in the display region for a patient, the area where the background color changes is larger (including not only the third sub-region, but also the first sub-region), which can further highlight the display region where the alarm is currently occurring, that is, the patient who is currently triggering the alarm is further highlighted, making it easier for the medical staff to pay attention to the alarm information that has not been confirmed.

Here, the third color and the first color may be the same color or different colors. In an example, the first color and the third color are the same color and are both the color capable of reflecting the level of the alarm information. For example, it is assumed that the alarm information is categorized as the high-level alarm information and the medium-level alarm information. The color corresponding to the high-level alarm information is red, and the color corresponding to the medium-level alarm information is yellow. Then, when the alarm information is the high-level alarm information, the high-level alarm information may be displayed in the third sub-region, and at the same time, the background colors of the first sub-region and the third sub-region may be changed from black to red, to enable the medical staff to immediately know that the high-level alarm has occurred upon they see the background color. Further, not only the background color of the third sub-region for displaying the alarm information reflects the color of the alarm level, but also the background color of the first sub-region reflects the color of the alarm level, thus the occurring and unconfirmed alarm is more prominent and it is easier to identify which patient the alarm originated from.

Similarly, when the alarm information is the medium-level alarm information, the medium-level alarm information may be displayed in the third sub-region, and at the same time, the background colors of the first sub-region and the third sub-region may be changed from black to yellow, to enable the medical staff to immediately know that the medium-level alarm has occurred upon they see the background color. Further, not only the background color of the third sub-region for displaying the alarm information reflects the color of the alarm level, but also the background color of the first sub-region reflects the color of the alarm level, thus the occurring and unconfirmed alarm is more prominent and it is easier to identify which patient the alarm originated from. It should be understood that the above-described colors are all exemplary, and in practical applications, the above-described colors may be set according to needs, and are not limited to the colors in the above-described examples.

With continued reference to FIG. 5, after the alarm information is confirmed, the background color of the third sub-region changes from the first color to the second color. It is assumed that the first color is red, the second color may be a dark red that becomes dim after being lowered in brightness, or a light red that becomes less prominent than before because it becomes more transparent after being lowered in transparency. In this way, the confirmed alarm information is displayed in a weakened manner relative to the alarm information before confirmation, and its interference to the medical staff will be greatly reduced. In addition, the background color of the first sub-region is also changed from the third color back to the original background color, so that the display difference before and after the alarm information is confirmed is greater, and it is more beneficial for the medical staff to distinguish the unconfirmed alarm information from the confirmed alarm information, thereby paying more attention to the alarm information that has not been confirmed.

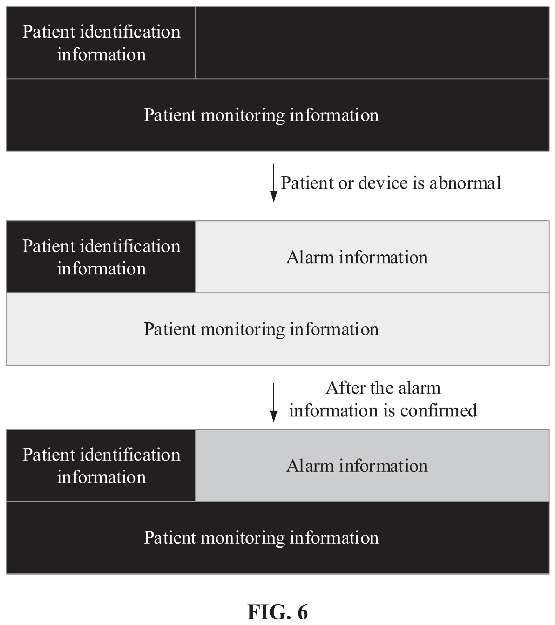

FIG. 6 illustrates another example diagram of displaying an additional prompt element on a hospital device according to an embodiment of the present disclosure, in which a schematic diagram of displaying the additional prompt element in a second sub-region is illustrated, and in this example, it is illustrated that the background color of the entire second sub-region is changed. It should be understood that the background color of a portion of the second sub-region may also be changed. As shown in FIG. 6, for example, before the alarm information is displayed, the background colors of the first sub-region, the second sub-region, and the third sub-region are all black (that is, the original background color), and when the alarm information is displayed, the background color of the third sub-region is changed, for example, from the original black to a more conspicuous first color (such as red or yellow), in addition to displaying the specific content of the alarm information (for example, text information such as high blood oxygen value) in the third sub-region. In this way, the alarm information may be more highlighted to attract the attention of the medical staff. In addition, the background color of the second sub-region is changed to a third color (for example, red or yellow, etc.) to further highlight which patient is currently triggering the alarm. Since the second sub-region is typically used for displaying the patient monitoring information, which generally includes various waveform data and numerical data, the second sub-region has a larger area compared to the first sub-region. At this time, in the display region for a patient, the area where the background color changes is larger (including not only the third sub-region, but also the larger second sub-region), which can further highlight the display region where the alarm is currently occurring, that is, the patient who is currently triggering the alarm is further highlighted, making it easier for the medical staff to pay attention to the alarm information that has not been confirmed.

Similar to the preceding description, the third color and the first color may be the same color or different colors. In an example, the first color and the third color are the same color and are both the color capable of reflecting the level of the alarm information. For example, it is assumed that the alarm information is categorized as the high-level alarm information and the medium-level alarm information. The color corresponding to the high-level alarm information is red, and the color corresponding to the medium-level alarm information is yellow. Then, when the alarm information is the high-level alarm information, the high-level alarm information may be displayed in the third sub-region, and at the same time, the background colors of the second sub-region and the third sub-region may be changed from black to red, to enable the medical staff to immediately know that the high-level alarm has occurred upon they see the background color. Further, not only the background color of the third sub-region for displaying the alarm information reflects the color of the alarm level, but also the background color of the second sub-region reflects the color of the alarm level, thus the occurring and unconfirmed alarm is more prominent and it is easier to identify which patient the alarm originated from.

Similarly, when the alarm information is the medium-level alarm information, the medium-level alarm information may be displayed in the third sub-region, and at the same time, the background colors of the second sub-region and the third sub-region may be changed from black to yellow, to enable the medical staff to immediately know that the medium-level alarm has occurred upon they see the background color. Further, not only the background color of the third sub-region for displaying the alarm information reflects the color of the alarm level, but also the background color of the second sub-region reflects the color of the alarm level, thus the occurring and unconfirmed alarm is more prominent and it is easier to identify which patient the alarm originated from. It should be understood that the above-described colors are all exemplary, and in practical applications, the above-described colors may be set according to needs, and are not limited to the colors in the above-described examples.

With continued reference to FIG. 6, after the alarm information is confirmed, the background color of the third sub-region changes from the first color to the second color. It is assumed that the first color is red, the second color may be a dark red that becomes dim after being lowered in brightness, or a light red that becomes less prominent than before because it becomes more transparent after being lowered in transparency. In this way, the confirmed alarm information is displayed in a weakened manner relative to the alarm information before confirmation, and its interference to the medical staff will be greatly reduced. In addition, the background color of the second sub-region is also changed from the third color back to the original background color, so that the display difference before and after the alarm information is confirmed is greater, and it is more beneficial for the medical staff to distinguish the unconfirmed alarm information from the confirmed alarm information, thereby paying more attention to the alarm information that has not been confirmed.

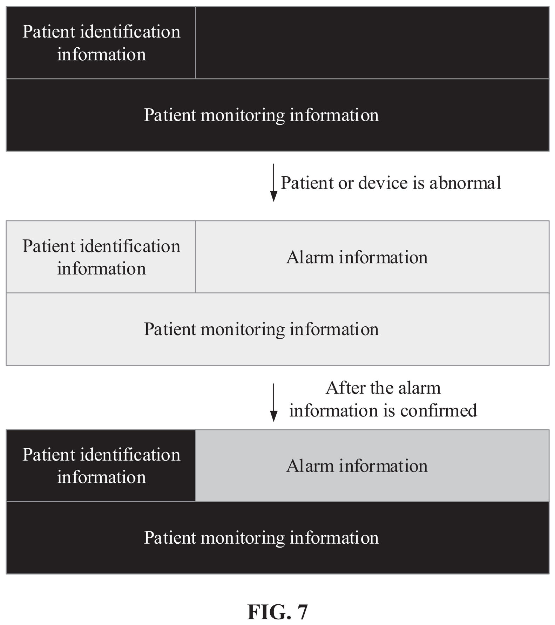

FIG. 7 illustrates another example diagram of displaying an additional prompt element on a hospital device according to an embodiment of the present disclosure, in which a schematic diagram of displaying the additional prompt element in first and second sub-regions is illustrated, and in this example, it is illustrated that the background colors of the entire first and second sub-regions are changed. It should be understood that the background color of a portion of the first and second sub-regions may also be changed. As shown in FIG. 7, for example, before the alarm information is displayed, the background colors of the first sub-region, the second sub-region, and the third sub-region are all black (that is, the original background color), and when the alarm information is displayed, the background color of the third sub-region is changed, for example, from the original black to a more conspicuous first color (such as red or yellow), in addition to displaying the specific content of the alarm information (for example, text information such as high blood oxygen value) in the third sub-region. In this way, the alarm information may be more highlighted to attract the attention of the medical staff. In addition, the background colors of the first and second sub-regions are changed to a third color (for example, red or yellow, etc.). This enables the background color of nearly the entire display region corresponding to the patient experiencing an abnormality to be changed, to further highlight the display region where the alarm is currently occurring, that is, the patient who is currently triggering the alarm is further highlighted, making it easier for the medical staff to pay attention to the alarm information that has not been confirmed.

Similar to the preceding description, the third color and the first color may be the same color or different colors. In an example, the first color and the third color are the same color and are both the color capable of reflecting the level of the alarm information. For example, it is assumed that the alarm information is categorized as the high-level alarm information and the medium-level alarm information. The color corresponding to the high-level alarm information is red, and the color corresponding to the medium-level alarm information is yellow. Then, when the alarm information is the high-level alarm information, the high-level alarm information may be displayed in the third sub-region, and at the same time, the background colors of the first sub-region, the second sub-region and the third sub-region may be changed from black to red, to enable the medical staff to immediately know that the high-level alarm has occurred upon they see the background color. Further, not only the background color of the third sub-region for displaying the alarm information reflects the color of the alarm level, but also the background color of nearly the entire display region corresponding to the patient experiencing an abnormality reflects the color of the alarm level, thus the occurring and unconfirmed alarm is more prominent and it is easier to identify which patient the alarm originated from.

Similarly, when the alarm information is the medium-level alarm information, the medium-level alarm information may be displayed in the third sub-region, and at the same time, the background colors of the first sub-region, the second sub-region and the third sub-region may be changed from black to yellow, to enable the medical staff to immediately know that the medium-level alarm has occurred upon they see the background color. Further, not only the background color of the third sub-region for displaying the alarm information reflects the color of the alarm level, but also the background color of nearly the entire display region corresponding to the patient experiencing an abnormality reflects the color of the alarm level, thus the occurring and unconfirmed alarm is more prominent and it is easier to identify which patient the alarm originated from. It should be understood that the above-described colors are all exemplary, and in practical applications, the above-described colors may be set according to needs, and are not limited to the colors in the above-described examples.

With continued reference to FIG. 7, after the alarm information is confirmed, the background color of the third sub-region changes from the first color to the second color. It is assumed that the first color is red, the second color may be a dark red that becomes dim after being lowered in brightness, or a light red that becomes less prominent than before because it becomes more transparent after being lowered in transparency. In this way, the confirmed alarm information is displayed in a weakened manner relative to the alarm information before confirmation, and its interference to the medical staff will be greatly reduced. In addition, the background colors of the first and second sub-regions are also changed from the third color back to the original background color, so that the display difference before and after the alarm information is confirmed is greater, and it is more beneficial for the medical staff to distinguish the unconfirmed alarm information from the confirmed alarm information, thereby paying more attention to the alarm information that has not been confirmed.

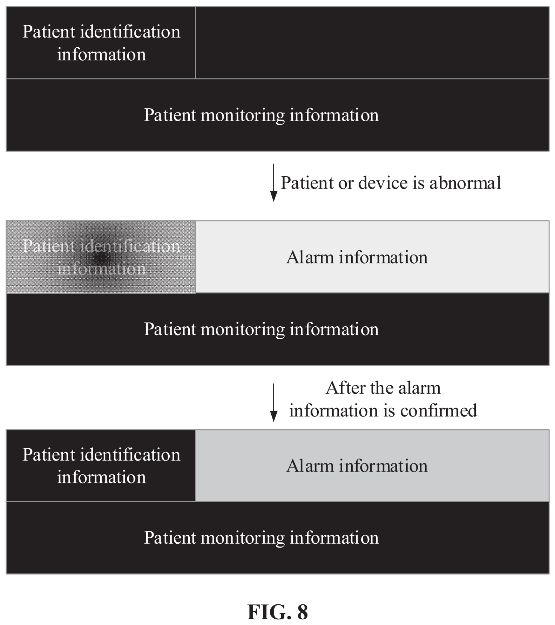

In another embodiment of the present disclosure, the above-described operation of displaying the additional prompt element while displaying the alarm information in the first display mode may include: adding a display layer within at least one portion of the first sub-region (such as the first sub-region R1 shown in FIG. 2) and/or at least one portion of the second sub-region (such as the second sub-region R2 shown in FIG. 2). Accordingly, the operation of eliminating the additional prompt element while displaying the alarm information in the second display mode may include: deleting the added display layer. This will be described below with reference to FIG. 8 to FIG. 10.

FIG. 8 illustrates yet another example diagram of displaying an additional prompt element on a hospital device according to an embodiment of the present disclosure, in which a schematic diagram of displaying the additional prompt element in a first sub-region is illustrated, and an example in which a display layer is added in the first sub-region and the display layer covers the entire first sub-region is illustrated. It should be understood that the added display layer may also cover a portion of the first sub-region. As shown in FIG. 8, for example, before the alarm information is displayed, the first sub-region includes a background layer displaying the background color and a layer displaying the patient identification information. When the alarm information is displayed, the background color of the third sub-region is changed, for example, from the original black to a more conspicuous first color (such as red or yellow), in addition to displaying the specific content of the alarm information (for example, text information such as high blood oxygen value) in the third sub-region. In this way, the alarm information may be more highlighted to attract the attention of the medical staff. In addition, a display layer is added (such as adding one display layer) in the first sub-region, which may present a solid color shadow visual effect (similar to a background layer), a gradient shadow visual effect (for example, the color of the layer gradually fades from the periphery to the center, or vice versa, etc.), a banner pattern, or a logo pattern (such as a stripe pattern, a checkered pattern, etc.) to further highlight which patient is currently triggering the alarm. The first sub-region is generally used to display patient identification information, and the change of the display layer may further highlight the abnormal patient. In addition, in the display region for a patient, the area where the display mode is changed is larger (including not only the change in the background color of the third sub-region, but also the change in the display layer of the first sub-region), which can further highlight the display region where the alarm is currently occurring, that is, the patient who is currently triggering the alarm is further highlighted, making it easier for the medical staff to pay attention to the alarm information that has not been confirmed.

Here, the color of the added display layer and the first color may be the same color, similar colors, or different colors. In an example, the color of the added display layer and the first color are similar colors, and both are colors capable of reflecting the level of the alarm information. For example, it is assumed that the alarm information is categorized as high-level alarm information and medium-level alarm information. The color corresponding to the high-level alarm information is red, and the color corresponding to the medium-level alarm information is yellow. Then, when the alarm information is the high-level alarm information, the high-level alarm information may be displayed in the third sub-region, and at the same time, the background color of the third sub-region is changed from black to red, and a display layer is added in the first sub-region, for example, in a color that gradually fades from red from the periphery to the center, so that the medical staff may immediately know that the high-level alarm has occurred upon they see the background color. Further, not only the background color of the third sub-region for displaying the alarm information reflects the color of the alarm level, but also the color of the added display layer in the first sub-region reflects the color of the alarm level, thus the occurring and unconfirmed alarm is more prominent and it is easier to identify which patient the alarm originated from.

Similarly, when the alarm information is the medium-level alarm information, the medium-level alarm information may be displayed in the third sub-region, and at the same time, the background color of the third sub-region is changed from black to yellow, and a display layer is added in the first sub-region, for example, in a color that gradually fades from yellow from the periphery to the center, so that the medical staff can immediately know that the medium-level alarm has occurred upon they see the background color. Further, not only the background color of the third sub-region for displaying the alarm information reflects the color of the alarm level, but also the color of the added display layer in the first sub-region reflects the color of the alarm level, thus the occurring and unconfirmed alarm is more prominent and it is easier to identify which patient the alarm originated from. It should be understood that the above-described colors are all exemplary, and in practical applications, the above-described colors may be set according to needs, and are not limited to the colors in the above-described examples.

With continued reference to FIG. 8, after the alarm information is confirmed, the background color of the third sub-region changes from the first color to the second color. It is assumed that the first color is red, the second color may be a dark red that becomes dim after being lowered in brightness, or a light red that becomes less prominent than before because it becomes more transparent after being lowered in transparency. In this way, the confirmed alarm information is displayed in a weakened manner relative to the alarm information before confirmation, and its interference to the medical staff will be greatly reduced. In addition, the added display layer in the first sub-region has been eliminated and is no longer displayed, so that the display difference before and after the alarm information is confirmed is greater, and it is more beneficial for the medical staff to distinguish the unconfirmed alarm information from the confirmed alarm information, thereby paying more attention to the alarm information that has not been confirmed.

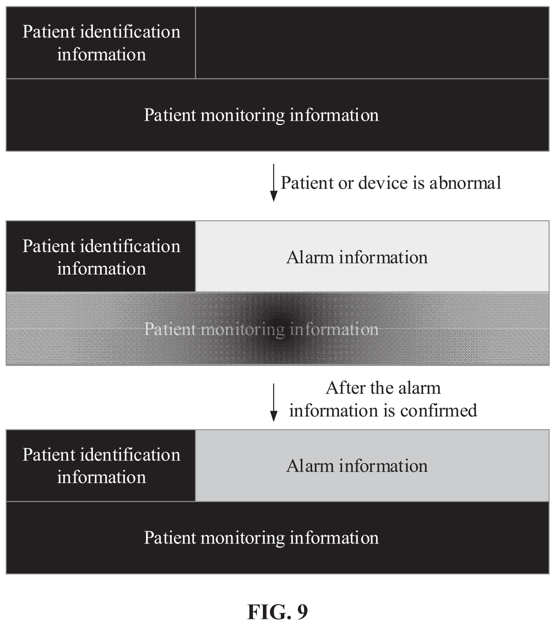

FIG. 9 illustrates yet another example diagram of displaying an additional prompt element on a hospital device according to an embodiment of the present disclosure, in which a schematic diagram of displaying the additional prompt element in a second sub-region is illustrated, and an example in which a display layer is added in the second sub-region and the display layer covers the entire second sub-region is illustrated. It should be understood that the added display layer may also cover a portion of the second sub-region. As shown in FIG. 9, for example, before the alarm information is displayed, the second sub-region includes a background layer displaying the background color and a layer displaying the patient monitoring information. When the alarm information is displayed, the background color of the third sub-region is changed, for example, from the original black to a more conspicuous first color (such as red or yellow), in addition to displaying the specific content of the alarm information (for example, text information such as high blood oxygen value) in the third sub-region. In this way, the alarm information may be more highlighted to attract the attention of the medical staff. In addition, a display layer is added (such as adding one display layer) in the second sub-region, which may present a solid color shadow visual effect (similar to a background layer), a gradient shadow visual effect (for example, the color of the layer gradually fades from the periphery to the center, or vice versa, etc.), a banner pattern, or a logo pattern (such as a stripe pattern, a checkered pattern, etc.), to further highlight which patient is currently triggering the alarm. Since the second sub-region is typically used for displaying the patient monitoring information, which generally includes various waveform data and numerical data, the second sub-region has a larger area compared to the first sub-region. At this time, in the display region for a patient, the area where the display mode is changed is larger (including not only the change in the background color of the third sub-region, but also the change in the display layer of the larger second sub-region), which can further highlight the display region where the alarm is currently occurring, that is, the patient who is currently triggering the alarm is further highlighted, making it easier for the medical staff to pay attention to the alarm information that has not been confirmed.

Here, the color of the added display layer and the first color may be the same color, similar colors, or different colors. In an example, the color of the added display layer and the first color are similar colors, and both are colors capable of reflecting the level of the alarm information. For example, it is assumed that the alarm information is categorized as high-level alarm information and medium-level alarm information. The color corresponding to the high-level alarm information is red, and the color corresponding to the medium-level alarm information is yellow. Then, when the alarm information is the high-level alarm information, the high-level alarm information may be displayed in the third sub-region, and at the same time, the background color of the third sub-region is changed from black to red, and a display layer is added in the second sub-region, for example, in a color that gradually fades from red from the periphery to the center, so that the medical staff may immediately know that the high-level alarm has occurred upon they see the background color. Further, not only the background color of the third sub-region for displaying the alarm information reflects the color of the alarm level, but also the color of the added display layer in the second sub-region reflects the color of the alarm level, thus the occurring and unconfirmed alarm is more prominent and it is easier to identify which patient the alarm originated from.

Similarly, when the alarm information is the medium-level alarm information, the medium-level alarm information may be displayed in the third sub-region, and at the same time, the background color of the third sub-region is changed from black to yellow, and a display layer is added in the second sub-region, for example, in a color that gradually fades from yellow from the periphery to the center, so that the medical staff can immediately know that the medium-level alarm has occurred upon they see the background color. Further, not only the background color of the third sub-region for displaying the alarm information reflects the color of the alarm level, but also the color of the added display layer in the second sub-region reflects the color of the alarm level, thus the occurring and unconfirmed alarm is more prominent and it is easier to identify which patient the alarm originated from. It should be understood that the above-described colors are all exemplary, and in practical applications, the above-described colors may be set according to needs, and are not limited to the colors in the above-described examples.

With continued reference to FIG. 9, after the alarm information is confirmed, the background color of the third sub-region changes from the first color to the second color. It is assumed that the first color is red, the second color may be a dark red that becomes dim after being lowered in brightness, or a light red that becomes less prominent than before because it becomes more transparent after being lowered in transparency. In this way, the confirmed alarm information is displayed in a weakened manner relative to the alarm information before confirmation, and its interference to the medical staff will be greatly reduced. In addition, the added display layer in the second sub-region has been eliminated and is no longer displayed, so that the display difference before and after the alarm information is confirmed is greater, and it is more beneficial for the medical staff to distinguish the unconfirmed alarm information from the confirmed alarm information, thereby paying more attention to the alarm information that has not been confirmed.

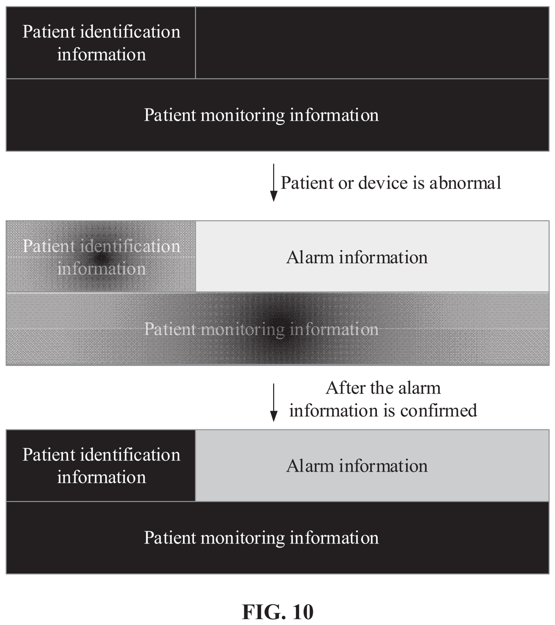

FIG. 10 illustrates yet another example diagram of displaying an additional prompt element on a hospital device according to an embodiment of the present disclosure, in which a schematic diagram of displaying the additional prompt element in first and second sub-regions is illustrated, and an example in which display layer(s) is(are) added in the first and second sub-regions and the display layer(s) covers(cover) the first and second sub-regions entirely is illustrated. It should be understood that the added display layer may also cover a portion of the first and second sub-regions. As shown in FIG. 10, for example, before the alarm information is displayed, the first sub-region includes a background layer displaying the background color and a layer displaying the patient identification information, and the second sub-region includes a background layer displaying the background color and a layer displaying the patient monitoring information. When the alarm information is displayed, the background color of the third sub-region is changed, for example, from the original black to a more conspicuous first color (such as red or yellow), in addition to displaying the specific content of the alarm information (for example, text information such as high blood oxygen value) in the third sub-region. In this way, the alarm information may be more highlighted to attract the attention of the medical staff. In addition, display layer(s) is(are) added in the first and second sub-regions (such as adding a display layer covering both the first and second sub-regions, or adding a respective display layer for each of the first and second sub-regions), which may present a solid color shadow visual effect (similar to a background layer), a gradient shadow visual effect (for example, the color of the layer gradually fades from the periphery to the center, or vice versa, etc.), a banner pattern, or a logo pattern (such as a stripe pattern, a checkered pattern, etc.). This causes the display mode in nearly the entire display region corresponding to the patient experiencing an abnormality to be changed (including not only the change in the background color of the third sub-region, but also the change in the display layer of the first and second sub-regions), which can further highlight the display region where the alarm is currently occurring, that is, the patient who is currently triggering the alarm is further highlighted, making it easier for the medical staff to pay attention to the alarm information that has not been confirmed.

Here, the color of the added display layer and the first color may be the same color, similar colors, or different colors. In an example, the color of the added display layer and the first color are similar colors, and both are colors capable of reflecting the level of the alarm information. For example, it is assumed that the alarm information is categorized as high-level alarm information and medium-level alarm information. The color corresponding to the high-level alarm information is red, and the color corresponding to the medium-level alarm information is yellow. Then, when the alarm information is the high-level alarm information, the high-level alarm information may be displayed in the third sub-region, and at the same time, the background color of the third sub-region is changed from black to red, and display layer(s) is(are) added in the first and second sub-regions, for example, in a color that gradually fades from red from the periphery to the center, so that the medical staff may immediately know that the high-level alarm has occurred upon they see the background color. Further, not only the background color of the third sub-region for displaying the alarm information reflects the color of the alarm level, but also the color of the added display layer(s) in the first and second sub-regions reflects the color of the alarm level, thus the occurring and unconfirmed alarm is more prominent and it is easier to identify which patient the alarm originated from.

Similarly, when the alarm information is the medium-level alarm information, the medium-level alarm information may be displayed in the third sub-region, and at the same time, the background color of the third sub-region is changed from black to yellow, and display layer(s) is(are) added in the first and second sub-regions, for example, in a color that gradually fades from yellow from the periphery to the center, so that the medical staff can immediately know that the medium-level alarm has occurred upon they see the background color. Further, not only the background color of the third sub-region for displaying the alarm information reflects the color of the alarm level, but also the color of the added display layer(s) in the first and second sub-regions reflects the color of the alarm level, thus the occurring and unconfirmed alarm is more prominent and it is easier to identify which patient the alarm originated from. It should be understood that the above-described colors are all exemplary, and in practical applications, the above-described colors may be set according to needs, and are not limited to the colors in the above-described examples.

With continued reference to FIG. 10, after the alarm information is confirmed, the background color of the third sub-region changes from the first color to the second color. It is assumed that the first color is red, the second color may be a dark red that becomes dim after being lowered in brightness, or a light red that becomes less prominent than before because it becomes more transparent after being lowered in transparency. In this way, the confirmed alarm information is displayed in a weakened manner relative to the alarm information before confirmation, and its interference to the medical staff will be greatly reduced. In addition, the added display layer(s) in the first and second sub-regions has(have) been eliminated and is(are) no longer displayed, so that the display difference before and after the alarm information is confirmed is greater, and it is more beneficial for the medical staff to distinguish the unconfirmed alarm information from the confirmed alarm information, thereby paying more attention to the alarm information that has not been confirmed.

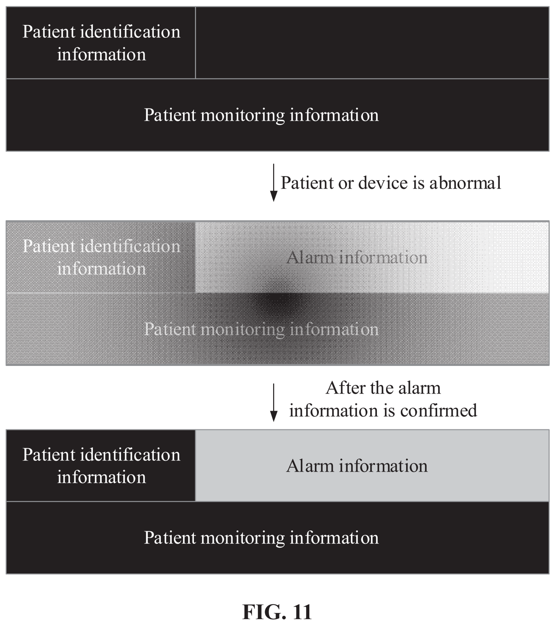

FIG. 11 illustrates yet another example diagram of displaying an additional prompt element on a hospital device according to an embodiment of the present disclosure, in which a schematic diagram of displaying the additional prompt element in the display region in its entirety for a patient experiencing an abnormality is illustrated, and an example in which a display layer is added in the display region for the patient experiencing an abnormality and the display layer covers the display region entirely is illustrated. As shown in FIG. 11, for example, before the alarm information is displayed, the first sub-region includes a background layer displaying the background color and a layer displaying the patient identification information, the second sub-region includes a background layer displaying the background color and a layer displaying the patient monitoring information, and the third sub-region includes a background layer displaying the background color and a layer displaying the patient alarm information. When the alarm information is displayed, the background color of the third sub-region is changed, for example, from the original black to a more conspicuous first color (such as red or yellow), in addition to displaying the specific content of the alarm information (for example, text information such as high blood oxygen value) in the third sub-region. In this way, the alarm information may be more highlighted to attract the attention of the medical staff. In addition, display layer(s) is(are) added in the first, second and third sub-regions (such as adding a display layer covering all of the first, second and third sub-regions, or adding a respective display layer for each of the first, second and third sub-regions), which may present a solid color shadow visual effect (similar to a background layer), a gradient shadow visual effect (for example, the color of the layer gradually fades from the periphery to the center, or vice versa, etc.), a banner pattern, or a logo pattern (such as a stripe pattern, a checkered pattern, etc.). This causes the display mode in nearly the entire display region corresponding to the patient experiencing an abnormality to be changed (including not only the change in the background color of the third sub-region, but also the change in the display layer of the entire display region), which can further highlight the display region where the alarm is currently occurring, that is, the patient who is currently triggering the alarm is further highlighted, making it easier for the medical staff to pay attention to the alarm information that has not been confirmed.

Here, the color of the added display layer and the first color may be the same color, similar colors, or different colors. In an example, the color of the added display layer and the first color are similar colors, and both are colors capable of reflecting the level of the alarm information. For example, it is assumed that the alarm information is categorized as high-level alarm information and medium-level alarm information. The color corresponding to the high-level alarm information is red, and the color corresponding to the medium-level alarm information is yellow. Then, when the alarm information is the high-level alarm information, the high-level alarm information may be displayed in the third sub-region, and at the same time, the background color of the third sub-region is changed from black to red, and a display layer is added into the display region in its entirety corresponding to the patient experiencing the abnormality, for example, in a color that gradually fades from red from the periphery to the center, so that the medical staff may immediately know that the high-level alarm has occurred upon they see the background color. Further, not only the background color of the third sub-region for displaying the alarm information reflects the color of the alarm level, but also the entire display region corresponding to the patient experiencing the abnormality is overlaid with a newly added display layer reflecting the color of the alarm level, thus the occurring and unconfirmed alarm is more prominent and it is easier to identify which patient the alarm originated from.

Similarly, when the alarm information is the medium-level alarm information, the medium-level alarm information may be displayed in the third sub-region, and at the same time, the background color of the third sub-region is changed from black to yellow, and a display layer is added into the display region in its entirety corresponding to the patient experiencing the abnormality, for example, in a color that gradually fades from yellow from the periphery to the center, so that the medical staff can immediately know that the medium-level alarm has occurred upon they see the background color. Further, not only the background color of the third sub-region for displaying the alarm information reflects the color of the alarm level, but also the entire display region corresponding to the patient experiencing the abnormality is overlaid with a newly added display layer reflecting the color of the alarm level, thus the occurring and unconfirmed alarm is more prominent and it is easier to identify which patient the alarm originated from. It should be understood that the above-described colors are all exemplary, and in practical applications, the above-described colors may be set according to needs, and are not limited to the colors in the above-described examples.

With continued reference to FIG. 11, after the alarm information is confirmed, the background color of the third sub-region changes from the first color to the second color. It is assumed that the first color is red, the second color may be a dark red that becomes dim after being lowered in brightness, or a light red that becomes less prominent than before because it becomes more transparent after being lowered in transparency. In this way, the confirmed alarm information is displayed in a weakened manner relative to the alarm information before confirmation, and its interference to the medical staff will be greatly reduced. In addition, the newly added display layer covering the entire display region has been eliminated and is no longer displayed, so that the display difference before and after the alarm information is confirmed is greater, and it is more beneficial for the medical staff to distinguish the unconfirmed alarm information from the confirmed alarm information, thereby paying more attention to the alarm information that has not been confirmed.

In the above examples, it is mainly described that the additional prompt element is displayed by changing a background color of at least one portion of the first sub-region and/or at least one portion of the second sub-region, or the additional prompt element is displayed by adding a display layer within the display region where the alarm information is present (the display layer covers at least one portion of the region outside the third sub-region or covers the display region in its entirety where the alarm information is present). In other examples, the additional prompt element may also be displayed in other ways. For example, a color of at least one display layer in at least one portion of the first sub-region and/or at least one portion of the second sub-region is changed to display the additional prompt element; a background color of the display region in its entirety where the alarm information is present is changed to display the additional prompt element; a color of a display layer in the display region in its entirety where the alarm information is present is changed to display the additional prompt element; or a display layer is added in the display region where the alarm information is present to display the additional prompt element, where the display layer covers the display region entirely, or the like, which is not described in detail herein. The foregoing methods may also be combined according to needs and feasibility.

In the above examples, the additional prompt element may reflect the level of the alarm information, for example, the changed background color described above (i.e., the background color of the display region occupied by the additional prompt element) reflects the level of the alarm information, and the color of the changed or added display layer (i.e., the color of the display layer presenting the additional prompt element) reflects the level of the alarm information. The level of the alarm information, as described above, includes for example the high-level alarm, the medium-level alarm and the like, and different alarm levels reflect different urgencies to be processed. In other examples, the additional prompt element may also reflect the type of the alarm information, for example, the changed background color described above reflects the type of the alarm information, and the color of the changed or added display layer reflects the type of the alarm information. The type of the alarm information, as described above, includes for example a physiological alarm and a technical alarm, and different alarm types reflect different alarm sources. Therefore, in the embodiments of the present disclosure, the operation of displaying the additional prompt element may include: displaying an additional prompt element corresponding to the alarm information. The respective additional prompt elements corresponding to at least two types of alarm information are different. For example, as previously described, different levels of alarm information correspond to different additional prompt elements displayed (such as different background colors changed in the previously described examples, and/or different colors of the changed or newly added display layers).

Regarding the physiological alarm information and the technical alarm information, as described above, the third sub-region R3 may be further divided into two sub-units, one sub-unit displays the technical alarm information and the other sub-unit displays the physiological alarm information. The background colors of the two sub-units may be the same or different. For example, the background color of the sub-unit corresponding to the higher-level alarm information in the physiological alarm information and the technical alarm information is closer to a background color of the region where the additional prompt element is present or to a color of a display layer presenting the additional prompt element, than the background color of the sub-unit corresponding to the lower-level alarm information in the physiological alarm information and the technical alarm information. More specific examples will be given below.

In an example, the third sub-region in the display region for a patient includes two sub-units, the first sub-unit displays the physiological alarm information and the second sub-unit displays the technical alarm information. The currently displayed physiological alarm information is high-level alarm information and the technical alarm information is medium-level alarm information. The background color of the first sub-unit where the physiological alarm information is present is changed from the original background color (i.e., black) to red, which reflects the high-level alarm information, and the background color of the second sub-unit where the technical alarm information is present is changed from the original background color (i.e., black) to yellow, which reflects the medium-level alarm information. At this time, to better highlight the presence of the high-level alarm for the patient, the additional prompt element is displayed, such as adding a display layer with a gradual red shadow to the entire display region for the patient. In this example, the background color of the first sub-suit is closer to the color of the added display layer than the second sub-suit. Here, the proximity of colors may be quantified, for example, by calculating RGB values to obtain close colors.

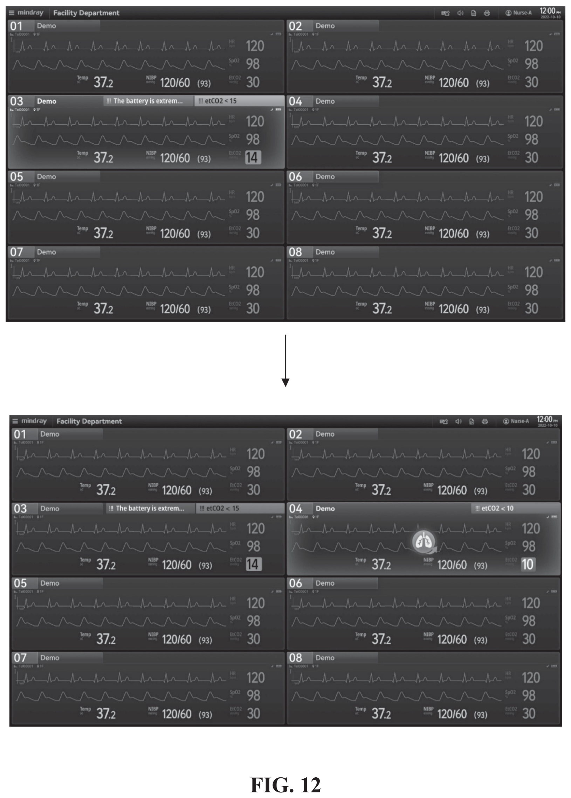

The first display mode, the second display mode and the additional prompt element are presented in schematic form in the above examples. An example of the first display mode, the second display mode and the additional prompt element is presented more specifically below with reference to FIG. 12 by using an interface diagram as an example.

As shown in FIG. 12, patient information for eight patients is displayed on the display interface, so the display interface is divided into display regions arranged in four rows and two columns, and each display region displays the patient information for one patient. The displayed patient information includes at least monitoring information such as a waveform diagram and a numerical value. In addition, the display region for patient 03 displays two pieces of alarm information, one is the technical alarm information related to a battery and the other is the physiological alarm information related to carbon dioxide (CO2). As can be seen from the figure, the background color of the sub-region where the alarm information is displayed is more prominent than the color of other regions, which may highlight the occurrence of the alarm information. In addition, the display region for the patient 03 is added with a display layer (an inwardly expanded shadow) covering the display region entirely, to further highlight the patient who triggers the alarm information.

With continued reference to FIG. 12, after the alarm information is confirmed, the display layer for the patient 03 disappears from the interface, and the background color of the sub-region for displaying the alarm information becomes dim (displayed in a weakened manner), to prevent distracting the attention of the application user from the new alarm. On the interface after the alarm confirmation for the patient 03 is confirmed, new alarm information occurs in the display region for patient 04, which is the physiological alarm information related to CO2. As can be seen from the figure, the background color of the sub-region where the alarm information is displayed is more prominent than the color of other regions, which may highlight the occurrence of alarm information. In addition, the display region for the patient 04 is added with a display layer (an inwardly expanded shadow) covering the display region entirely, to further highlight the patient who triggers the alarm information.

With continued reference to FIG. 12, a lung icon is also displayed in the display region for the patient 04, which indicates that a respiratory system of the patient is abnormal. In an embodiment of the present disclosure, when a body system of the patient is abnormal, the patient information may further include system abnormality information, and the display displays the system abnormality information, which may include: displaying an icon corresponding to the system indicated by the system abnormality information. For example, when the respiratory system of the patient is abnormal, a small lung icon may be displayed in the display region for the patient, as shown in FIG. 12, to prompt the user that the respiratory system of the patient is abnormal. When a confirmation operation for the system abnormality information (the icon) is received, the display may no longer display the icon. Alternatively, when no confirmation operation for the system abnormality information is received within a preset time period, the display also no longer displays the icon. This is because the system abnormality of the patient usually requires a long time to recover gradually, and if the confirmation operation for the system abnormality information has not been received for a long time, it may be assumed that the user has seen the icon and performed appropriate processing. The icon is still displayed only because the system abnormality of the patient has not yet completely recovered, but the abnormality has been processed and there is no need to display it again.

The hospital device according to an embodiment of the present disclosure is illustratively shown above. Based on the above description, the hospital device according to the embodiment of the present disclosure displays the patient information for a plurality of patients on the same display interface. Before receiving the confirmation operation for the alarm information, the alarm information is displayed in a relatively prominent first display mode, and the additional prompt elements is additionally displayed, which is beneficial for the medical staff to notice the alarm information and process it in time. After receiving the confirmation operation for the alarm information, the alarm information is displayed in a less prominent and visually weakened second display mode, and the previously displayed additional prompt element is eliminated, to prevent the alarm information from being too conspicuous and distracting the attention of the medical staff from other unconfirmed alarm information, which enables the medical staff to more easily notice other unconfirmed alarm information.

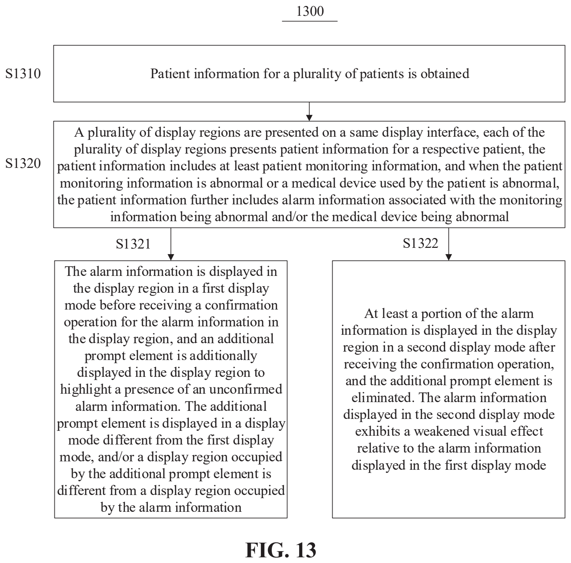

An information display method 1300 for a hospital device provided by another aspect of the present disclosure is described below with reference to FIG. 13. The method 1300 is applied to the hospital device 100 described above. The manner in which the hospital device 100 displays information has been described in detail above, and those skilled in the art may understand the specific details of the information display method 1300 for the hospital device in conjunction with the above description. For the sake of brevity, only some main operations of the information display method 1300 for the hospital device are described herein, and specific details will not be repeated.

As shown in FIG. 13, the information display method 1300 for the hospital device includes the following operations.

At S1310, patient information for a plurality of patients is obtained.

At S1320, a plurality of display regions are presented on a same display interface, and each of the plurality of display regions presents the patient information for a respective patient. The patient information includes at least patient monitoring information, and when the patient monitoring information is abnormal or a medical device used by the patient is abnormal, the patient information further includes alarm information associated with the monitoring information being abnormal and/or the medical device being abnormal.

For at least one display region where the alarm information is present, the operation of displaying the alarm information in S1320 may further include operations S1321 and S1322.

At S1321, the alarm information is displayed in the display region in a first display mode before receiving a confirmation operation for the alarm information in the display region, and an additional prompt element is additionally displayed in the display region to highlight a presence of an unconfirmed alarm information. The additional prompt element is displayed in a display mode different from the first display mode, and/or a display region occupied by the additional prompt element is different from the display region occupied by the alarm information.

At S1322, at least a portion of the alarm information is displayed in the display region in a second display mode after receiving the confirmation operation, and the additional prompt element is eliminated. The alarm information displayed in the second display mode exhibits a weakened visual effect relative to the alarm information displayed in the first display mode.

In an embodiment of the present disclosure, the patient information further includes patient identification information, and each of the plurality of display regions includes a first sub-region for displaying the patient identification information, a second sub-region for displaying the patient monitoring information, and a third sub-region for displaying the alarm information.

In an embodiment of the present disclosure, the first display mode includes: changing a background color of the third sub-region from an original background color to a first color; and the second display mode includes: changing the background color of the third sub-region from the first color to a second color. The second color is obtained by reducing a brightness or transparency of the first color.

In an embodiment of the present disclosure, the first display mode includes: displaying the alarm information with a first text attribute; and the second display mode includes: displaying the alarm information with a second text attribute. The first text attribute has a higher brightness of a text color and/or a bolder text font than the second text attribute.

In an embodiment of the present disclosure, the operation of additionally displaying the additional prompt element may include: changing a background color of at least one portion of the first sub-region and/or at least one portion of the second sub-region from an original background color to a third color. The operation of eliminating the additional prompt element may include: changing the background color of the at least one portion of the first sub-region and/or the at least one portion of the second sub-region from the third color back to the original background color.

In an embodiment of the present disclosure, the operation of additionally displaying the additional prompt element may include: adding a display layer within at least one portion of the first sub-region and/or at least one portion of the second sub-region. The operation of eliminating the additional prompt element may include: deleting the added display layer.

In an embodiment of the present disclosure, the display layer exhibits one of: a solid color shadow visual effect, a gradient shadow visual effect, a banner pattern, or a logo pattern.

In an embodiment of the present disclosure, the operation of additionally displaying the additional prompt element may include: displaying an additional prompt element corresponding to the alarm information. The respective additional prompt elements corresponding to at least two types of alarm information are different.

In an embodiment of the present disclosure, the patient information further includes patient identification information, and each of the plurality of display regions includes a first sub-region for displaying the patient identification information, a second sub-region for displaying the patient monitoring information, and a third sub-region for displaying the alarm information. The operation of additionally displaying the additional prompt element corresponding to the alarm information may include at least one of: changing a background color of at least one portion of the first sub-region and/or at least one portion of the second sub-region; changing a color of at least one display layer in at least one portion of the first sub-region and/or at least one portion of the second sub-region; or adding a display layer within at least one portion of the first sub-region and/or at least one portion of the second sub-region. The respective additional prompt elements corresponding to the at least two types of alarm information are different, including that: background colors of the display regions occupied by the respective additional prompt elements corresponding to the at least two types of alarm information are different; and/or colors of the display layers presenting the respective additional prompt elements corresponding to the at least two types of alarm information are different.

In an embodiment of the present disclosure, the operation of additionally displaying the additional prompt element corresponding to the alarm information may include at least one of: changing a background color of the display region in its entirety where the alarm information is present; changing a color of a display layer in the display region in its entirety where the alarm information is present; or adding a display layer in the display region where the alarm information is present, where the display layer covers the display region entirely. The respective additional prompt elements corresponding to the at least two types of alarm information are different, including that: background colors of the display regions occupied by the respective additional prompt elements corresponding to the at least two types of alarm information are different; and/or colors of the display layers presenting the respective additional prompt elements corresponding to the at least two types of alarm information are different.

In an embodiment of the present disclosure, the background color of the display region occupied by the additional prompt element reflects a level of the alarm information; the color of the display layer presenting the additional prompt element reflects the level of the alarm information.

In an embodiment of the present disclosure, the alarm information includes physiological alarm information of the patient and technical alarm information related to the device used by the patient. The third sub-region is configured to display the physiological alarm information or the technical alarm information, whichever has a higher level, or the third sub-region is divided into two sub-units for displaying the physiological alarm information and the technical alarm information respectively.

In an embodiment of the present disclosure, background colors of the two sub-units are different, and the background color of the sub-unit corresponding to the higher-level alarm information in the physiological alarm information and the technical alarm information is closer to a background color of the region where the additional prompt element is present or to a color of a display layer presenting the additional prompt element, than the background color of the sub-unit corresponding to the lower-level alarm information in the physiological alarm information and the technical alarm information.

In an embodiment of the present disclosure, the patient information further includes system abnormality information when a body system of the patient is abnormal, and the system abnormality information is displayed, including: displaying an icon corresponding to a system indicated by the system abnormality information; and stopping displaying the icon, when a confirmation operation for the system abnormality information is received, or when no confirmation operation for the system abnormality information is received within a preset time period.

Based on the above description, the information display method 1300 for the hospital device according to the embodiment of the present disclosure displays the patient information for a plurality of patients on the same display interface. Before receiving the confirmation operation for the alarm information, the alarm information is displayed in a relatively prominent first display mode, and the additional prompt elements is additionally displayed, which is beneficial for the medical staff to notice the alarm information and process it in time. After receiving the confirmation operation for the alarm information, the alarm information is displayed in a less prominent and visually weakened second display mode, and the previously displayed additional prompt element is eliminated, to prevent the alarm information from being too conspicuous and distracting the attention of the medical staff from other unconfirmed alarm information, which enables the medical staff to more easily notice other unconfirmed alarm information.

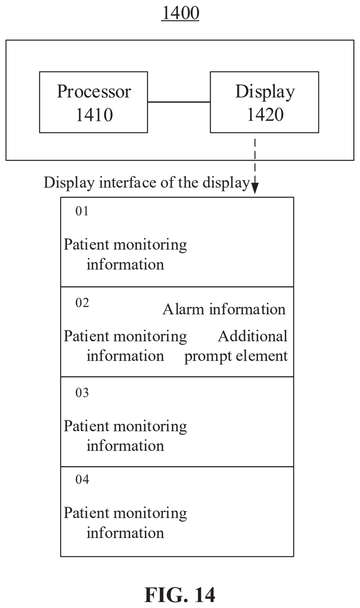

A hospital device 1400 provided by yet another aspect of the present disclosure is described below in conjunction with FIG. 14. As shown in FIG. 14, the hospital device 1400 includes a processor 1410 and a display 1420.

The processor 1410 is configured to obtain patient information for a plurality of patients. For example, the hospital device 1400 is communicatively connected with a medical device (such as a monitor, a ventilator, etc.) used by each of the plurality of patients, the processor 1410 may obtain monitoring information for each of the plurality of patients from these monitoring devices, and display the monitoring information on the display 1420. Further, when the patient monitoring information is abnormal or the medical device used by the patient is abnormal, alarm information, associated with the monitoring information being abnormal and/or the medical device being abnormal, may be displayed on the display 1420, and the alarm information is also one type of patient information.

The display 1420 is configured to present a plurality of display regions, such as display region 01, display region 02, display region 03, and display region 04 shown in FIG. 1, on a same display interface, and each of the plurality of display regions presents the patient information for a respective patient. For the sake of brevity, FIG. 1 illustrates only four display regions on the display interface for presenting patient information for four patients, which is merely an example. In practical applications, the number of display regions, and an arrangement of the number of rows and the number of columns included in the display interface may depend on the number of patients to be monitored, and the display interface may include one column and multiple rows, two columns and multiple rows, multiple columns and multiple rows, etc.

The patient information presented in each display region includes at least the patient monitoring information described above. When the patient monitoring information is abnormal or the medical device used by the patient is abnormal, the patient information further includes alarm information associated with the monitoring information being abnormal and/or the medical device being abnormal.

When the processor 1410 obtains the alarm information associated with the monitoring information being abnormal and/or the medical device being abnormal, the display 1420 is configured to: display the alarm information in a third sub-region (for example, the third sub-region R3 described above, or a sub-region at other positions of the display region where the alarm information is present) in a display region for the patient corresponding to the alarm information (FIG. 1 illustrates an example where the display region 02 is the display region containing the alarm information, and in practice, the number of display regions containing the alarm information may be one or more), and additionally display an additional prompt element corresponding to the alarm information in at least one portion of the region outside the third sub-region or in the display region in its entirety where the alarm information is present. The respective additional prompt elements corresponding to at least two types of alarm information are different.

In the embodiment of the present disclosure, the display 1420 of the hospital device 1400 displays the patient information for a plurality of patients on the same display interface. When the patient exhibits an abnormality (abnormal patient monitoring information or abnormal medical device used by the patient), the corresponding alarm information will be displayed in a certain sub-region in the display region for the patient, and the additional prompt element is additionally displayed in at least one portion of the region outside the sub-region or in the entire display region for the patient, to highlight the currently occurring alarm information, which makes it easier for the medical staff to notice the newly occurring alarm information and process it in time. Such a display scheme is particularly important for displaying information of multiple patients on the same interface, because there may be a situation where multiple patients are abnormal and multiple pieces of alarm information are displayed in different display regions in the same interface. Such a display mode not only displays the alarm information in a sub-region in the display region for the abnormal patient, but also displays the additional prompt element in other sub-regions or even the entire region of the display region for the abnormal patient, which is more beneficial for the medical staff to pay attention to the newly occurred alarm information and the patient corresponding to the alarm information. Moreover, the additional prompt element corresponds to (the type, level, etc. of) the alarm information, which enables the medical staff not only to easily notice the newly occurring alarm information and the patient corresponding to the alarm information, but also to intuitively understand whether the alarm is urgent, thereby avoiding an emergency alarm from being overlooked due to numerous alarm information on the interface, and thus avoiding serious consequences.

The hospital device 1400 according to the embodiment of the present disclosure is similar to the hospital device 100 according to the embodiment of the present disclosure described above, that is, when the alarm information is displayed, an additional prompt element is displayed to highlight the currently occurring alarm information. The difference between the two hospital devices is that: the hospital device 1400 according to the embodiment of the present disclosure does not define the change in the display mode before and after the alarm information is confirmed. In addition, in the hospital device 1400 according to the embodiment of the present disclosure, the displayed additional prompt element corresponds to the alarm information, and the displayed additional prompt elements corresponding to different alarm information (for example, different types or different levels) are also different, which is implemented in similar ways in the above various embodiments of the hospital device 100. Therefore, the hospital device 1400 according to the embodiment of the present disclosure is generally similar to the hospital device 100 according to the embodiment of the present disclosure described above, except for the different emphasis. In the various embodiments described below, most of the content is also similar to that of the previous embodiments. For the sake of brevity, the content is briefly described below and the details will not be repeated.

In an embodiment of the present disclosure, the display 1420 displaying the additional prompt element corresponding to the alarm information in the at least one portion of the region outside the third sub-region, includes at least one of: changing a background color of the at least one portion of the region outside the third sub-region; changing a color of at least one display layer in the at least one portion of the region outside the third sub-region; or adding a display layer in the at least one portion of the region outside the third sub-region.

In an embodiment of the present disclosure, the display 1420 additionally displaying the additional prompt element corresponding to the alarm information in the display region in its entirety where the alarm information is present, includes at least one of: changing a background color of the display region in its entirety where the alarm information is present; changing a color of a display layer in the display region in its entirety where the alarm information is present; or adding a display layer in the display region where the alarm information is present, where the display layer covers the display region entirely.

In an embodiment of the present disclosure, the respective additional prompt elements corresponding to the at least two types of alarm information being different, includes that: background colors of the display regions occupied by the respective additional prompt elements corresponding to the at least two types of alarm information are different; and/or colors of the display layers presenting the respective additional prompt elements corresponding to the at least two types of alarm information are different.

In an embodiment of the present disclosure, the background color of the display region occupied by the additional prompt element reflects a level of the alarm information; the color of the display layer presenting the additional prompt element reflects the level of the alarm information.

In an embodiment of the present disclosure, the display layer presents one of: a solid color shadow visual effect, a gradient color shadow visual effect, a banner pattern, or a logo pattern.

In an embodiment of the present disclosure, the alarm information includes physiological alarm information of the patient and technical alarm information related to the device used by the patient. The third sub-region is configured to display the physiological alarm information or the technical alarm information, whichever has a higher level, or the third sub-region is divided into two sub-units for displaying the physiological alarm information and the technical alarm information respectively.

In an embodiment of the present disclosure, background colors of the two sub-units are different, and the background color of the sub-unit corresponding to the higher-level alarm information in the physiological alarm information and the technical alarm information is closer to a background color of the region where the additional prompt element is present or to a color of a display layer presenting the additional prompt element, than the background color of the sub-unit corresponding to the lower-level alarm information in the physiological alarm information and the technical alarm information.

In an embodiment of the present disclosure, the processor 1410 is further configured to eliminate the additional prompt element after receiving a confirmation operation for the alarm information.

In an embodiment of the present disclosure, the processor 1410 eliminating the additional prompt element, includes at least one of: changing the background color of the display region occupied by the additional prompt element back to an original background color; changing the color of the display layer presenting the additional prompt element back to an original color; or deleting the display layer presenting the additional prompt element.

In an embodiment of the present disclosure, the display 1420 displaying the alarm information, further includes: displaying the alarm information in the third sub-region in a first display mode before receiving a confirmation operation for the alarm information; and displaying at least a portion of the alarm information in the third sub-region in a second display mode after receiving the confirmation operation, and eliminating the additional prompt element. The alarm information displayed in the second display mode exhibits a weakened visual effect relative to the alarm information displayed in the first display mode.

In an embodiment of the present disclosure, the first display mode includes: changing a background color of the third sub-region from an original background color to a first color; and the second display mode includes: changing the background color of the third sub-region from the first color to a second color. The second color is obtained by reducing a brightness or transparency of the first color.

In an embodiment of the present disclosure, the first display mode includes: displaying the alarm information with a first text attribute; and the second display mode includes: displaying the alarm information with a second text attribute. The first text attribute has a higher brightness of a text color and/or a bolder text font than the second text attribute.

In an embodiment of the present disclosure, the patient information further includes system abnormality information when a body system of the patient is abnormal, and the display 1420 is further configured to display the system abnormality information, including the display further configured to: display an icon corresponding to a system indicated by the system abnormality information; and stop displaying the icon by the display 1420, when a confirmation operation for the system abnormality information is received, or when no confirmation operation for the system abnormality information is received within a preset time period.

Based on the above description, the hospital device 1400 according to the embodiment of the present disclosure displays the patient information for a plurality of patients on the same display interface. When the patient exhibits an abnormality, the corresponding alarm information will be displayed in a certain sub-region in the display region for the patient, and the additional prompt element will be additionally displayed in at least one portion of the region outside the sub-region or in the entire display region for the patient to highlight the currently occurring alarm information, which makes it easier for the medical staff to notice the newly occurring alarm information and process it in time. Moreover, the displayed additional prompt element corresponds to (the type, level, etc. of) the alarm information, which enables the medical staff not only to easily notice the newly occurring alarm information and the patient corresponding to the alarm information, but also to intuitively understand whether the alarm is urgent, thereby avoiding an emergency alarm from being overlooked due to numerous alarm information on the interface, and thus avoiding serious consequences.

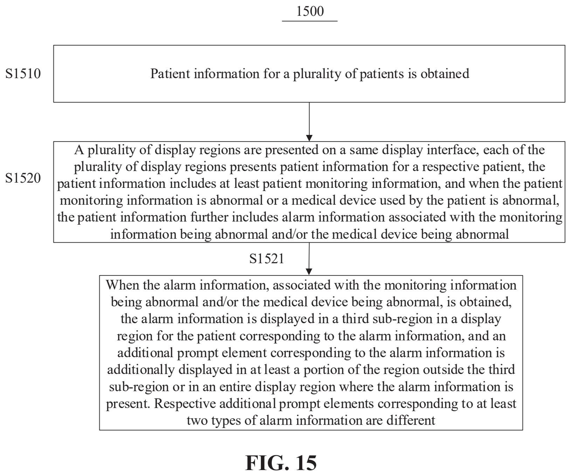

An information display method 1500 for a hospital device provided by another aspect of the present disclosure is described below in conjunction with FIG. 15, and the method 1500 is applied to the hospital device 1400 described above, in which specific details of the information display method 1500 for the hospital device 1400 have been described in detail. For the sake of brevity, only some main operations of the information display method 1500 for the hospital device are described herein, and specific details will not be repeated.

As shown in FIG. 15, the information display method 1500 for the hospital device includes the following operations.

At S1510, patient information for a plurality of patients is obtained.

At S1520, a plurality of display regions are presented on a same display interface, and each of the plurality of display regions presents patient information for a respective patient. The patient information includes at least patient monitoring information, and when the patient monitoring information is abnormal or a medical device used by the patient is abnormal, the patient information further includes alarm information associated with the monitoring information being abnormal and/or the medical device being abnormal.

When the alarm information, associated with the monitoring information being abnormal and/or the medical device being abnormal, is obtained, the operation of displaying the alarm information in S1520 may further include operation S1521.

At S1521, when the alarm information, associated with the monitoring information being abnormal and/or the medical device being abnormal, is obtained, the alarm information is displayed in a third sub-region in a display region for the patient corresponding to the alarm information, and an additional prompt element corresponding to the alarm information is additionally displayed in at least one portion of the region outside the third sub-region or in the display region in its entirety where the alarm information is present. Respective additional prompt elements corresponding to at least two types of alarm information are different.

In an embodiment of the present disclosure, the operation of displaying the additional prompt element corresponding to the alarm information in the at least one portion of the region outside the third sub-region includes at least one of: changing a background color of the at least one portion of the region outside the third sub-region; changing a color of at least one display layer in the at least one portion of the region outside the third sub-region; or adding a display layer in the at least one portion of the region outside the third sub-region.

In an embodiment of the present disclosure, the operation of additionally displaying the additional prompt element corresponding to the alarm information in the display region in its entirety where the alarm information is present includes at least one of: changing a background color of the display region in its entirety where the alarm information is present; changing a color of a display layer in the display region in its entirety where the alarm information is present; or adding a display layer in the display region where the alarm information is present, where the display layer covers the display region entirely.

In an embodiment of the present disclosure, the respective additional prompt elements corresponding to the at least two types of alarm information being different, includes that: background colors of the display regions occupied by the respective additional prompt elements corresponding to the at least two types of alarm information are different; and/or colors of the display layers presenting the respective additional prompt elements corresponding to the at least two types of alarm information are different.

In an embodiment of the present disclosure, the background color of the display region occupied by the additional prompt element reflects a level of the alarm information; and/or the color of the display layer presenting the additional prompt element reflects the level of the alarm information.

In an embodiment of the present disclosure, the display layer presents one of: a solid color shadow visual effect, a gradient color shadow visual effect, a banner pattern, or a logo pattern.

In an embodiment of the present disclosure, the alarm information includes physiological alarm information of the patient and technical alarm information related to the device used by the patient. The third sub-region is configured to display the physiological alarm information or the technical alarm information, whichever has a higher level, or the third sub-region is divided into two sub-units for displaying the physiological alarm information and the technical alarm information respectively.

In an embodiment of the present disclosure, background colors of the two sub-units are different, and the background color of the sub-unit corresponding to the higher-level alarm information in the physiological alarm information and the technical alarm information is closer to a background color of the region where the additional prompt element is present or to the color of the display layer presenting the additional prompt element, than the background color of the sub-unit corresponding to the lower-level alarm information in the physiological alarm information and the technical alarm information.

In an embodiment of the present disclosure, the method further includes: eliminating the additional prompt element after receiving a confirmation operation for the alarm information.

In an embodiment of the present disclosure, the operation of eliminating the additional prompt element includes at least one of: changing the background color of the display region occupied by the additional prompt element back to an original background color; changing the color of the display layer presenting the additional prompt element back to an original color; or deleting the display layer presenting the additional prompt element.

In an embodiment of the present disclosure, the operation of displaying the alarm information includes: displaying the alarm information in the third sub-region in a first display mode before receiving a confirmation operation for the alarm information; and displaying at least a portion of the alarm information in the third sub-region in a second display mode after receiving the confirmation operation, and eliminating the additional prompt element. The alarm information displayed in the second display mode exhibits a weakened visual effect relative to the alarm information displayed in the first display mode.

In an embodiment of the present disclosure, the first display mode includes: changing a background color of the third sub-region from an original background color to a first color; and the second display mode includes: changing the background color of the third sub-region from the first color to a second color. The second color is obtained by reducing a brightness or transparency of the first color.

In an embodiment of the present disclosure, the first display mode includes: displaying the alarm information with a first text attribute; and the second display mode includes: displaying the alarm information with a second text attribute. The first text attribute has a higher brightness of a text color and/or a bolder text font than the second text attribute.

In an embodiment of the present disclosure, the patient information further includes system abnormality information when a body system of the patient is abnormal, and the system abnormality information is displayed, including: displaying an icon corresponding to a system indicated by the system abnormality information; and stopping displaying the icon, when a confirmation operation for the system abnormality information is received, or when no confirmation operation for the system abnormality information is received within a preset time period.

Based on the above description, the information display method 1500 for the hospital device according to the embodiment of the present disclosure displays the patient information for a plurality of patients on the same display interface. When the patient exhibits an abnormality, the corresponding alarm information will be displayed in a certain sub-region in the display region for the patient, and the additional prompt element will be additionally displayed in at least one portion of the region outside the sub-region or in the entire display region for the patient to highlight the currently occurring alarm information, which makes it easier for the medical staff to notice the newly occurring alarm information and process it in time. Moreover, the displayed additional prompt element corresponds to (the type, level, etc. of) the alarm information, which enables the medical staff not only to easily notice the newly occurring alarm information and the patient corresponding to the alarm information, but also to intuitively understand whether the alarm is urgent, thereby avoiding an emergency alarm from being overlooked due to numerous alarm information on the interface, and thus avoiding serious consequences.

Further, in an embodiment of the present disclosure, there is further provided a storage medium for storing program instructions that, when executed by a computer or a processor, cause the computer or the processor to perform corresponding operations of the information display method for the hospital device according to the embodiments of the present disclosure. The storage medium may include, for example, a memory card in a smartphone, a storage component in a tablet computer, a hard disk in a personal computer, a read-only memory (ROM), an erasable programmable ROM (EPROM), a portable compact disk ROM (CD-ROM), a USB memory, or any combination of the above storage media. The computer-readable storage medium may be any combination of one or more computer-readable storage media.

Although exemplary embodiments have been described herein with reference to the accompanying drawings, it should be understood that the above-described embodiments are merely exemplary and are not intended to limit the scope of the present disclosure thereto. Various changes and modifications may be made by those of ordinary skill in the art without departing from the scope and spirit of the present disclosure. All such changes and modifications are intended to be included within the scope of the present disclosure as claimed by the appended claims.

Those of ordinary skill in the art will appreciate that the elements and algorithmic steps of the various examples described in connection with the embodiments disclosed herein may be implemented in electronic hardware, or a combination of computer software and electronic hardware. Whether these functions are executed in hardware or software depends on the specific application and design constraints of the technical solutions. Those skilled in the art may use different methods for implementing the described functions for each particular application, but such implementations should not be considered beyond the scope of the present disclosure.

In several embodiments provided by the present disclosure, it should be understood that the disclosed device and method may be implemented in other ways. For example, the device embodiments described above are merely schematic. For example, the division of units is only a logical function division. In an actual implementation, there may be other division methods. For example, multiple units or components may be combined, or may be integrated into another device, or some features may be ignored, or may not be implemented.

In the specification provided herein, numerous specific details are set forth. However, it is to be understood that the embodiments of the present disclosure may be practiced without these specific details. In some examples, well-known methods, structures, and techniques are not described in detail to avoid obscuring the understanding of the present specification.

Similarly, it should be understood that in the description of the exemplary embodiments of the present disclosure, various features of the present disclosure are sometimes grouped together into a single embodiment, figure, or description thereof, in order to simplify the present disclosure and assist in understanding one or more of the various inventive aspects. However, the methods in the present disclosure should not be interpreted to reflect the intention that the claimed application claims more features than those explicitly recited in the claims. Rather, as reflected in the corresponding claims, the inventive point lies in solving the corresponding technical problem with features fewer than all of the features of a single disclosed embodiment. Accordingly, the claims that follow the detailed description are hereby expressly incorporated into the detailed description, with the claims themselves as separate embodiments of the present disclosure.

It will be understood by those skilled in the art that all features and all processes or elements of any method or apparatus so disclosed in this description (including the accompanying claims, abstract, and drawings) may be combined in any combination except that the features are mutually exclusive. Unless expressly stated otherwise, features disclosed in this description (including the accompanying claims, abstract, and drawings) may be replaced by alternative features that provide the same, equivalent, or similar purposes.

Furthermore, those skilled in the art will appreciate that while some embodiments described herein include certain features included in other embodiments rather than other features, combinations of features in different embodiments are meant to be within the scope of the present disclosure and form different embodiments. For example, in the claims, any of the claimed embodiments may be used in any combination.

The various component in the embodiments of the present disclosure may be implemented in hardware, or in software modules running on one or more processors, or in combinations thereof. Those skilled in the art will appreciate that a microprocessor or a digital signal processor (DSP) may be used in practice to implement some or all of the functionality of some modules according to the embodiments of the present disclosure. The present disclosure may also be implemented as a program (e.g., a computer program and a computer program product) for performing some or all of the methods described herein. Such a program implementing the present disclosure may be stored on a computer-readable storage medium, or may have the form of one or more signals. Such signals may be downloaded from an Internet site, or provided on a carrier signal, or provided in any other form.

It should be noted that the above-described embodiments illustrate the present disclosure rather than limit the present disclosure, and that those skilled in the art may design alternative embodiments without departing from the scope of the appended claims. In the claims, any reference numbers placed between parentheses should not be construed as limiting the claims. The present disclosure may be implemented by means of hardware including several different elements and by means of a suitably programmed computer. In a unit claim enumerating several devices, several of these devices may be specifically embodied by the same item of hardware. The use of the words "first", "second", "third", etc. does not indicate any order, and these words may be interpreted as names.

The foregoing description pertains solely to specific embodiments of the present disclosure or explanations of the specific embodiments, and the scope of protection of the present disclosure is not limited thereto. Changes or substitutions easily conceived by any person skilled in the art within the technical scope disclosed in the present disclosure should be covered within the scope of protection of the present disclosure. The scope of protection of the present disclosure should be subject to the scope of protection of the claims.

## Claims

1. A hospital device, comprising a processor and a display, wherein
the processor is configured to obtain patient information for a plurality of patients; and
the display is configured to present a plurality of display regions on a same display interface, each of the plurality of display regions presents the patient information for a respective patient, the patient information comprises at least patient monitoring information, and when the patient monitoring information is abnormal or a medical device used by the patient is abnormal, the patient information further comprises alarm information associated with the monitoring information being abnormal and/or the medical device being abnormal;
wherein for at least one display region where the alarm information is present,
displaying the alarm information in said display region in a first display mode before receiving a confirmation operation for the alarm information in said display region, and additionally displaying an additional prompt element in said display region to highlight a presence of an unconfirmed alarm information, wherein the additional prompt element is displayed in a display mode different from the first display mode, and/or a display region occupied by the additional prompt element is different from a display region occupied by the alarm information; and
displaying at least a portion of the alarm information in said display region in a second display mode after receiving the confirmation operation, and eliminating the additional prompt element, wherein the alarm information displayed in the second display mode exhibits a weakened visual effect relative to the alarm information displayed in the first display mode.

2. The hospital device of claim 1, wherein the patient information further comprises patient identification information, each of the plurality of display regions comprises a first sub-region for displaying the patient identification information, a second sub-region for displaying the patient monitoring information, and a third sub-region for displaying the alarm information.

3. The hospital device of claim 2, wherein
the first display mode comprises: changing a background color of the third sub-region from an original background color to a first color; and
the second display mode comprises: changing the background color of the third sub-region from the first color to a second color, wherein the second color is obtained by reducing a brightness or transparency of the first color.

4. The hospital device of claim 1, wherein
the first display mode comprises: displaying the alarm information with a first text attribute; and
the second display mode comprises: displaying the alarm information with a second text attribute;
wherein the first text attribute has a higher brightness of a text color and/or a bolder text font than the second text attribute.

5. The hospital device of claim 2, wherein
additionally displaying an additional prompt element comprises: changing a background color of at least one portion of the first sub-region and/or at least one portion of the second sub-region from an original background color to a third color; and
eliminating the additional prompt element comprises: changing the background color of the at least one portion of the first sub-region and/or the at least one portion of the second sub-region from the third color back to the original background color.

6. The hospital device of claim 2, wherein
additionally displaying an additional prompt element comprises: adding a display layer within at least one portion of the first sub-region and/or at least one portion of the second sub-region; and
eliminating the additional prompt element comprises: deleting the added display layer.

7. The hospital device of claim 6, wherein the display layer exhibits one of: a solid color shadow visual effect, a gradient shadow visual effect, a banner pattern, or a logo pattern.

8. The hospital device of claim 1, wherein
additionally displaying an additional prompt element comprises: displaying an additional prompt element corresponding to the alarm information,
wherein respective additional prompt elements corresponding to at least two types of alarm information are different.

9. The hospital device of claim 8, wherein the patient information further comprises patient identification information, each of the plurality of display regions comprises a first sub-region for displaying the patient identification information, a second sub-region for displaying the patient monitoring information, and a third sub-region for displaying the alarm information;
additionally displaying an additional prompt element corresponding to the alarm information comprises at least one of:
changing a background color of at least one portion of the first sub-region and/or at least one portion of the second sub-region;
changing a color of at least one display layer in at least one portion of the first sub-region and/or at least one portion of the second sub-region; or
adding a display layer within at least one portion of the first sub-region and/or at least one portion of the second sub-region,
respective additional prompt elements corresponding to the at least two types of alarm information being different, comprises that: background colors of the display regions occupied by the respective additional prompt elements corresponding to the at least two types of alarm information are different; and/or colors of the display layers presenting the respective additional prompt elements corresponding to the at least two types of alarm information are different.

10. The hospital device of claim 8, wherein additionally displaying an additional prompt element corresponding to the alarm information comprises at least one of:
changing a background color of the display region in its entirety where the alarm information is present;
changing a color of a display layer in the display region in its entirety where the alarm information is present; or
adding a display layer in the display region where the alarm information is present, wherein the display layer covers the display region entirely;
respective additional prompt elements corresponding to the at least two types of alarm information being different, comprises that: background colors of the display regions occupied by the respective additional prompt elements corresponding to the at least two types of alarm information are different; and/or colors of the display layers presenting the respective additional prompt elements corresponding to the at least two types of alarm information are different.

11. The hospital device of claim 9 or 10, wherein
the background color of the display region occupied by the additional prompt element reflects a level of the alarm information; and/or
the color of the display layer presenting the additional prompt element reflects a level of the alarm information.

12. The hospital device of claim 2 or 9, wherein the alarm information comprises physiological alarm information of the patient and technical alarm information related to the device used by the patient, the third sub-region is configured to display the physiological alarm information or the technical alarm information, whichever has a higher level, or the third sub-region is divided into two sub-units for displaying the physiological alarm information and the technical alarm information respectively.

13. The hospital device of claim 12, wherein background colors of the two sub-units are different, and the background color of the sub-unit corresponding to the higher-level alarm information in the physiological alarm information and the technical alarm information is closer to a background color of the region where the additional prompt element is present or to a color of a display layer presenting the additional prompt element, than the background color of the sub-unit corresponding to the lower-level alarm information in the physiological alarm information and the technical alarm information.

14. The hospital device of any one of claims 1 to 10, wherein the patient information further comprises system abnormality information when a body system of the patient is abnormal, and the display is further configured to display the system abnormality information, comprising the display further configured to:
display an icon corresponding to a system indicated by the system abnormality information; and
stop displaying the icon by the display, when a confirmation operation for the system abnormality information is received, or when no confirmation operation for the system abnormality information is received within a preset time period.

15. An information display method for a hospital device, comprising:
obtaining patient information for a plurality of patients; and
presenting a plurality of display regions on a same display interface, each of the plurality of display regions presenting the patient information for a respective patient, the patient information comprising at least patient monitoring information, and when the patient monitoring information is abnormal or a medical device used by the patient is abnormal, the patient information further comprising alarm information associated with the monitoring information being abnormal and/or the medical device being abnormal;
for at least one display region where the alarm information is present,
displaying the alarm information in said display region in a first display mode before receiving a confirmation operation for the alarm information in said display region, and additionally displaying an additional prompt element in said display region to highlight a presence of an unconfirmed alarm information, wherein the additional prompt element is displayed in a display mode different from the first display mode, and/or a display region occupied by the additional prompt element is different from a display region occupied by the alarm information; and
displaying at least a portion of the alarm information in said display region in a second display mode after receiving the confirmation operation, and eliminating the additional prompt element, wherein the alarm information displayed in the second display mode exhibits a weakened visual effect relative to the alarm information displayed in the first display mode.

16. The method of claim 15, wherein the patient information further comprises patient identification information, and each of the plurality of display regions comprises a first sub-region for displaying the patient identification information, a second sub-region for displaying the patient monitoring information, and a third sub-region for displaying the alarm information.

17. The method of claim 16, wherein
the first display mode comprises: changing a background color of the third sub-region from an original background color to a first color; and
the second display mode comprises: changing the background color of the third sub-region from the first color to a second color, wherein the second color is obtained by reducing a brightness or transparency of the first color.

18. The method of claim 15, wherein
the first display mode comprises: displaying the alarm information with a first text attribute; and
the second display mode comprises: displaying the alarm information with a second text attribute;
the first text attribute has a higher brightness of a text color and/or a bolder text font than the second text attribute.

19. The method of claim 16, wherein
additionally displaying an additional prompt element comprises: changing a background color of at least one portion of the first sub-region and/or at least one portion of the second sub-region from an original background color to a third color; and
eliminating the additional prompt element comprises: changing the background color of the at least one portion of the first sub-region and/or the at least one portion of the second sub-region from the third color back to the original background color.

20. The method of claim 16, wherein
additionally displaying an additional prompt element comprises: adding a display layer within at least one portion of the first sub-region and/or at least one portion of the second sub-region; and
eliminating the additional prompt element comprises: deleting the added display layer.

21. The method of claim 20, wherein the display layer exhibits one of: a solid color shadow visual effect, a gradient shadow visual effect, a banner pattern, or a logo pattern.

22. The method of claim 15, wherein
additionally displaying an additional prompt element comprises: displaying an additional prompt element corresponding to the alarm information,
wherein respective additional prompt elements corresponding to at least two types of alarm information are different.

23. The method of claim 22, wherein the patient information further comprises patient identification information, each of the plurality of display regions comprising a first sub-region for displaying the patient identification information, a second sub-region for displaying the patient monitoring information, and a third sub-region for displaying the alarm information;
additionally displaying an additional prompt element corresponding to the alarm information comprises at least one of:
changing a background color of at least one portion of the first sub-region and/or at least one portion of the second sub-region;
changing a color of at least one display layer in at least one portion of the first sub-region and/or at least one portion of the second sub-region; or
adding a display layer within at least one portion of the first sub-region and/or at least one portion of the second sub-region,
respective additional prompt elements corresponding to the at least two types of alarm information being different, comprises that: background colors of the display regions occupied by the respective additional prompt elements corresponding to the at least two types of alarm information are different; and/or colors of the display layers presenting the respective additional prompt elements corresponding to the at least two types of alarm information are different.

24. The method of claim 22, wherein additionally displaying an additional prompt element corresponding to the alarm information comprises at least one of:
changing a background color of the display region in its entirety where the alarm information is present;
changing a color of a display layer in the display region in its entirety where the alarm information is present; or
adding a display layer in the display region where the alarm information is present, wherein the display layer covers the display region entirely;
respective additional prompt elements corresponding to the at least two types of alarm information being different, comprises that: background colors of the display regions occupied by the respective additional prompt elements corresponding to the at least two types of alarm information are different; and/or colors of the display layers presenting the respective additional prompt elements corresponding to the at least two types of alarm information are different.

25. The method of claim 23 or 24, wherein
the background color of the display region occupied by the additional prompt element reflects a level of the alarm information; and/or
the color of the display layer presenting the additional prompt element reflects a level of the alarm information.

26. The method of claim 16 or 23, wherein the alarm information comprises physiological alarm information of the patient and technical alarm information related to the device used by the patient, the third sub-region is configured to display the physiological alarm information or the technical alarm information, whichever has a higher level, or the third sub-region is divided into two sub-units for displaying the physiological alarm information and the technical alarm information respectively.

27. The method of claim 26, wherein background colors of the two sub-units are different, and the background color of the sub-unit corresponding to the higher-level alarm information in the physiological alarm information and the technical alarm information is closer to a background color of the region where the additional prompt element is present or to a color of a display layer presenting the additional prompt element, than the background color of the sub-unit corresponding to the lower-level alarm information in the physiological alarm information and the technical alarm information.

28. The method of any one of claims 15 to 24, wherein the patient information further comprises system abnormality information when a body system of the patient is abnormal, and displaying the system abnormality information comprises:
displaying an icon corresponding to a system indicated by the system abnormality information; and
stopping displaying the icon, when a confirmation operation for the system abnormality information is received, or when no confirmation operation for the system abnormality information is received within a preset time period.

29. A hospital device, comprising a processor and a display, wherein
the processor is configured to obtain patient information for a plurality of patients; and
the display is configured to present a plurality of display regions on a same display interface, each of the plurality of display regions presents the patient information for a respective patient, the patient information comprises at least patient monitoring information, and when the patient monitoring information is abnormal or a medical device used by the patient is abnormal, the patient information further comprises alarm information associated with the monitoring information being abnormal and/or the medical device being abnormal;
wherein when the processor obtains the alarm information associated with the monitoring information being abnormal and/or the medical device being abnormal, the display is further configured to: display the alarm information in a third sub-region in a display region for the patient corresponding to the alarm information, and additionally display an additional prompt element corresponding to the alarm information in at least one portion of the region outside the third sub-region or in the display region in its entirety where the alarm information is present;
wherein respective additional prompt elements corresponding to at least two types of alarm information are different.

30. The hospital device of claim 29, wherein the display further configured to display an additional prompt element corresponding to the alarm information in at least one portion of the region outside the third sub-region, comprises at least one of: the display further configured to:
change a background color of the at least one portion of the region outside the third sub-region;
change a color of at least one display layer in the at least one portion of the region outside the third sub-region; or
add a display layer in the at least one portion of the region outside the third sub-region.

31. The hospital device of claim 29, wherein the display further configured to additionally display an additional prompt element corresponding to the alarm information in the display region in its entirety where the alarm information is present, comprises at least one of: the display further configured to:
change a background color of the display region in its entirety where the alarm information is present;
change a color of a display layer in the display region in its entirety where the alarm information is present; or
add a display layer in the display region where the alarm information is present, wherein the display layer covers the display region entirely.

32. The hospital device of claim 30 or 31, wherein respective additional prompt elements corresponding to the at least two types of alarm information being different, comprises that:
background colors of the display regions occupied by the respective additional prompt elements corresponding to the at least two types of alarm information are different; and/or colors of the display layers presenting the respective additional prompt elements corresponding to the at least two types of alarm information are different.

33. The hospital device of claim 32, wherein
the background color of the display region occupied by the additional prompt element reflects a level of the alarm information; and/or
the color of the display layer presenting the additional prompt element reflects a level of the alarm information.

34. The hospital device of claim 31, wherein the display layer presents one of: a solid color shadow visual effect, a gradient color shadow visual effect, a banner pattern, or a logo pattern.

35. The hospital device of any one of claims 29 to 31, wherein the alarm information comprises physiological alarm information of the patient and technical alarm information related to the device used by the patient, the third sub-region is configured to display the physiological alarm information or the technical alarm information, whichever has a higher level, or the third sub-region is divided into two sub-units for displaying the physiological alarm information and the technical alarm information respectively.

36. The hospital device of claim 35, wherein background colors of the two sub-units are different, and the background color of the sub-unit corresponding to the higher-level alarm information in the physiological alarm information and the technical alarm information is closer to a background color of the region where the additional prompt element is present or to a color of a display layer presenting the additional prompt element, than the background color of the sub-unit corresponding to the lower-level alarm information in the physiological alarm information and the technical alarm information.

37. The hospital device of claim 30 or 31, wherein the processor is further configured to:
eliminate the additional prompt element after receiving a confirmation operation for the alarm information.

38. The hospital device of claim 37, wherein the processor further configured to eliminate the additional prompt element, comprises at least one of: the processor further configured to:
change the background color of the display region occupied by the additional prompt element back to an original background color;
change the color of the display layer presenting the additional prompt element back to an original color; or
delete the display layer presenting the additional prompt element.

39. The hospital device of any one of claims 29 to 31, wherein the display further configured to display the alarm information, comprises the display further configured to:
display the alarm information in the third sub-region in a first display mode before receiving a confirmation operation for the alarm information; and
display at least a portion of the alarm information in the third sub-region in a second display mode after receiving the confirmation operation, and eliminate the additional prompt element, wherein the alarm information displayed in the second display mode exhibits a weakened visual effect relative to the alarm information displayed in the first display mode.

40. The hospital device of claim 39, wherein
the first display mode comprises: changing a background color of the third sub-region from an original background color to a first color; and
the second display mode comprises: changing the background color of the third sub-region from the first color to a second color, wherein the second color is obtained by reducing a brightness or transparency of the first color.

41. The hospital device of claim 39, wherein
the first display mode comprises: displaying the alarm information with a first text attribute; and
the second display mode comprises: displaying the alarm information with a second text attribute;
wherein the first text attribute has a higher brightness of a text color and/or a bolder text font than the second text attribute.

42. The hospital device of any one of claims 29 to 31, wherein the patient information further comprises system abnormality information when a body system of the patient is abnormal, and the display is further configured to display the system abnormality information, comprising the display further configured to:
display an icon corresponding to a system indicated by the system abnormality information; and
stop displaying the icon by the display, when a confirmation operation for the system abnormality information is received, or when no confirmation operation for the system abnormality information is received within a preset time period.

43. An information display method for a hospital device, comprising:
obtaining patient information for a plurality of patients;
presenting a plurality of display regions on a same display interface, each of the plurality of display regions presenting the patient information for a respective patient, the patient information comprising at least patient monitoring information, and when the patient monitoring information is abnormal or a medical device used by the patient is abnormal, the patient information further comprising alarm information associated with the monitoring information being abnormal and/or the medical device being abnormal; and
when the alarm information, associated with the monitoring information being abnormal and/or the medical device being abnormal, is obtained, displaying the alarm information in a third sub-region in a display region for the patient corresponding to the alarm information, and additionally displaying an additional prompt element corresponding to the alarm information in at least one portion of the region outside the third sub-region or in the display region in its entirety where the alarm information is present;
wherein respective additional prompt elements corresponding to at least two types of alarm information are different.

44. The method of claim 43, wherein displaying an additional prompt element corresponding to the alarm information in the at least one portion of the region outside the third sub-region, comprises at least one of:
changing a background color of the at least one portion of the region outside the third sub-region;
changing a color of at least one display layer in the at least one portion of the region outside the third sub-region; or
adding a display layer in the at least one portion of the region outside the third sub-region.

45. The method of claim 43, wherein additionally displaying an additional prompt element corresponding to the alarm information in the display region in its entirety where the alarm information is present, comprises at least one of:
changing a background color of the display region in its entirety where the alarm information is present;
changing a color of a display layer in the display region in its entirety where the alarm information is present; or
adding a display layer in the display region where the alarm information is present, wherein the display layer covers the display region entirely.

46. The method of claim 44 or 45, wherein respective additional prompt elements corresponding to the at least two types of alarm information being different, comprises that:
background colors of the display regions occupied by the respective additional prompt elements corresponding to the at least two types of alarm information are different; and/or colors of the display layers presenting the respective additional prompt elements corresponding to the at least two types of alarm information are different.

47. The method of claim 46, wherein
the background color of the display region occupied by the additional prompt element reflects a level of the alarm information; and/or
the color of the display layer presenting the additional prompt element reflects a level of the alarm information.

48. The method of claim 45, wherein the display layer presents one of: a solid color shadow visual effect, a gradient color shadow visual effect, a banner pattern, or a logo pattern.

49. The method of any one of claims 43 to 45, wherein the alarm information comprises physiological alarm information of the patient and technical alarm information related to the device used by the patient, and the method further comprises:
displaying, in the third sub-region, the physiological alarm information or the technical alarm information, whichever has a higher level, or
dividing the third sub-region into two sub-units for displaying the physiological alarm information and the technical alarm information respectively.

50. The method of claim 49, wherein background colors of the two sub-units are different, and the background color of the sub-unit corresponding to the higher-level alarm information in the physiological alarm information and the technical alarm information is closer to a background color of the region where the additional prompt element is present or to a color of a display layer presenting the additional prompt element, than the background color of the sub-unit corresponding to the lower-level alarm information in the physiological alarm information and the technical alarm information.

51. The method of claim 44 or 45, further comprising:
eliminating the additional prompt element after receiving a confirmation operation for the alarm information.

52. The method of claim 47, wherein eliminating the additional prompt element comprises at least one of:
changing the background color of the display region occupied by the additional prompt element back to an original background color;
changing the color of the display layer presenting the additional prompt element back to an original color; or
deleting the display layer presenting the additional prompt element.

53. The method of any one of claims 43 to 45, wherein displaying the alarm information comprises:
displaying the alarm information in the third sub-region in a first display mode before receiving a confirmation operation for the alarm information; and
displaying at least a portion of the alarm information in the third sub-region in a second display mode after receiving the confirmation operation, and eliminating the additional prompt element, wherein the alarm information displayed in the second display mode exhibits a weakened visual effect relative to the alarm information displayed in the first display mode.

54. The method of claim 53, wherein
the first display mode comprises: changing a background color of the third sub-region from an original background color to a first color; and
the second display mode comprises: changing the background color of the third sub-region from the first color to a second color, wherein the second color is obtained by reducing a brightness or transparency of the first color.

55. The method of claim 53, wherein
the first display mode comprises: displaying the alarm information with a first text attribute; and
the second display mode comprises: displaying the alarm information with a second text attribute;
wherein the first text attribute has a higher brightness of a text color and/or a bolder text font than the second text attribute.

56. The method of any one of claims 43 to 45, wherein the patient information further comprises system abnormality information when a body system of the patient is abnormal, and the method further comprises displaying the system abnormality information, comprising:
displaying an icon corresponding to a system indicated by the system abnormality information; and
stopping displaying the icon, when a confirmation operation for the system abnormality information is received, or when no confirmation operation for the system abnormality information is received within a preset time period.
